# EUROPÄISCHE PATENTSCHRIFT

(11) **EP 2 992 295 B1**
(45) Veröffentlichungstag und Bekanntmachung des Hinweises auf die Patenterteilung: **04.01.2017**
(21) Anmeldenummer: 14730731.8
(22) Anmeldetag: 30.04.2014
(51) Int. Cl.: G01B 11/24, A22B 5/00

(54) **VORRICHTUNG UND VERFAHREN ZUR KLASSIFIKATION EINES LEBENSMITTELOBJEKTS GEWACHSENER ODER UNREGELMÄSSIGER STRUKTUR**
DEVICE AND METHOD FOR THE CLASSIFICATION OF A FOOD ITEM OF AN ORGANIC OR IRREGULAR STRUCTURE
DISPOSITIF ET PROCÉDÉ DE CLASSIFICATION D'UN OBJET ALIMENTAIRE DE STRUCTURE NATURELLE OU IRRÉGULIÈRE

(30) Priorität: 03.05.2013 DE 102013007531; 03.05.2013 DE 202013004094 U
(43) Veröffentlichungstag der Anmeldung: 09.03.2016
(73) Patentinhaber: CSB-System AG, 52511 Geilenkirchen (DE)
(72) Erfinder: SCHIMITZEK, Peter, 52511 Geilenkirchen (DE)
(74) Vertreter: Weihrauch, Frank
(86) Internationale Anmeldenummer: PCT/DE2014/000225
(87) Internationale Veröffentlichungsnummer: WO 2014/177131

(56) Entgegenhaltungen:
- EP-A2- 0 730 146
- DE-A1- 4 408 604
- US-A1- 2003 072 472

## Beschreibung

Die Erfindung betrifft eine Vorrichtung sowie ein Verfahren, insbesondere zur automatischen Klassifikation eines Lebensmittelobjekts anhand einer Bilderfassung.

Aus dem Stand der Technik ist es bereits bekannt, Lebensmittel, insbesondere Schlachttierkörper, manuell zu klassifizieren, indem das Lebensmittel beispielsweise auf oder an einer Fördereinrichtung an einem entsprechenden Arbeitsplatz, auch als Identifikationspunkt bezeichnet, vorbeigeführt wird und dabei, anhand seiner Merkmale, visuell durch eine zuständige Mitarbeiterin oder einen Mitarbeiter klassifiziert wird.
Hierzu sind ferner Vorrichtungen bekannt, welche eine Datenbank mit verschiedenartigen Bildern von Lebensmittelobjekten und eine Dateneingabevorrichtung aufweisen und mittels derer die Mitarbeiterin oder der Mitarbeiter das jeweilige Lebensmittel/Schlachttierkörperobjekt anhand der Bilder klassifizieren kann.

Die manuelle Klassifikation von Lebensmittelobjekten hat insbesondere den Nachteil, dass zum einen erhöhte Kosten durch die Bereitstellung eines entsprechenden Arbeitsplatzes verursacht werden und dass zum anderen die manuelle Klassifikation eines durch die menschliche Wahrnehmungs- und Reaktionsgeschwindigkeit bestimmten Zeitaufwandes bedarf.
Der Erhöhung des Durchsatzes der Lebensmittel oder Schlachttierkörperobjekte durch den Identifikationspunkt sind damit Grenzen gesetzt. Zudem würde mit einer Durchsatzerhöhung gleichzeitig die Gefahr von Fehlklassifikationen aufgrund menschlichen Versagens steigen.

Des Weiteren sind aus dem Stand der Technik bereits Lösungen bekannt, welche eine automatische Klassifikation von Waren anhand von Identifikationsträgern ermöglichen.

Solche Identifikationsträger können zum Beispiel Strichcodes oder RFID-Elemente sein, welche an der zu klassifizierenden Ware angebracht sind.
In diesem Zusammenhang besteht ein Nachteil insbesondere darin, dass letztlich bereits vor der Zuführung zu dem Identifikationspunkt eine manuelle Klassifikation durchgeführt werden muss, wobei deren Ergebnis die Basis für die Beschreibung der Identifikationsträger bildet. Ein weiterer Nachteil besteht in dem Zusatzaufwand für die Bereitstellung des Identifikationsträgers.

Weitere bekannte Vorrichtungen ermöglichen darüber hinaus auch eine automatische Klassifikation, insbesondere von Industriegütern, mittels einer Bilderfassung des jeweiligen Industrieguts und des Herausfilterns konkreter Gütermerkmale aus dem erfassten Bild.
Derartige Vorrichtungen können jedoch nur bei Industriegütern mit definierten und gleichbleibenden Merkmalen, beispielsweise Längenabmessungen, Farbe oder Oberflächenbeschaffenheit, zuverlässig eingesetzt werden.

Ein Verfahren zur Schlachttierkörperbewertung anhand selektierter Gewebekomponenten ist aus der Druckschrift DE 44 08 604 A1 bekannt. Dabei erfolgt eine Bildaufnahme des zu bewertenden Schlachttierkörperobjekts vor einem blauen Hintergrund und eine anschließende Digitalisierung und Speicherung der erfassten Bilder auf einem Computer. Anhand von zuvor bestimmten Farbklassifikationen und häufigen Farbwerten werden zum einen die äußeren Konturen des Schlachttierkörperobjekts bestimmt und zum anderen bestimmte Bildpunkte den jeweiligen Geweben zugeordnet. Darüber hinaus wird die Rückseite des Schlachttierkörpers zur dreidimensionalen Objektbestimmung, unter Einbindung eines Lichtschnittverfahrens, optisch erfasst, um hierdurch die Konformationsklasse des Schlachttierkörpers zu bewerten.

Des Weiteren wird in der Druckschrift EP 0 730 146 A2 ein Verfahren zur Bestimmung von Qualitätsmerkmalen eines Tierkörpers vorgeschlagen. Dieses Verfahren sieht eine erste Bilderfassung des Tierkörpers mittels einer Videokamera vor, wobei der Tierkörper während der ersten Bilderfassung von mehreren Lampen angestrahlt wird. Anschließend wird eine zweite Bilderfassung ohne Beleuchtung des Tierkörpers durchgeführt und aus den RGB-Abweichungen der beiden Bilderfassungsergebnisse die Qualitätsmerkmale des Tierkörpers bestimmt.

Ferner offenbart die Druckschrift US 2003/072472 A1 ein System zur Bewertung des Fleisches und zur Abschätzung des Fleischertrages bzw. der Fleischqualität an einem Schlachttier. Das System umfasst hierbei eine Bildkamera mit einer Beleuchtungsvorrichtung sowie eine Auswerte-einheit. Anhand eines erfassten Bildes spezifischer Sektionen des Schlachttieres werden die relevanten Merkmale wie Fettgehalt, Ribeye, Magerfleischanteil, etc. mittels Bildanalyse bestimmt.
Aufgabe der Erfindung ist es, eine Vorrichtung sowie ein Verfahren bereitzustellen, welche insbesondere eine selbstständig durchführbare Klassifikation eines Lebensmittelobjekts ermöglichen und welche dabei eine sichere Klassifikation mit einer hohen Erkennungsrate und -Sicherheit bei gleichzeitig hohem Objektdurchsatz ermöglichen.
Die Aufgabe wird in Bezug auf die Vorrichtung durch die im Patentanspruch 1 aufgeführten Merkmale und in Bezug auf das Verfahren durch die im Patentanspruch 8 aufgeführten Merkmale gelöst. Bevorzugte Weiterbildungen ergeben sich aus den jeweiligen abhängigen Ansprüchen.
Eine erfindungsgemäße Vorrichtung zur Klassifikation eines Lebensmittelobjekts gewachsener oder unregelmäßiger Struktur wird insbesondere an einem sogenannten Identifikationspunkt, beispielsweise als Teil einer Verarbeitungsstrecke in einem Schlachttierbetrieb, mit dem Ziel angewendet, das Lebensmittelobjekt zu klassifizieren und dieses einer konkreten Artikelnummer oder -bezeichnung zuzuordnen.
Als Lebensmittelobjekt gewachsener oder unregelmäßiger Struktur, nachfolgend verkürzt als Lebensmittelobjekt bezeichnet, wird im Sinne der erfindungsgemäßen technischen Lösung Folgendes verstanden: Lebensmittelobjekte gewachsener Struktur sind-Schlachttierkörperobjekte, welche als ganze Schlachttierkörper oder Teile hiervon, beispielsweise als Schweinehälften, Schinken und so weiter, vorliegen können, und pflanzliche Objekte, welche ebenfalls ganz oder in Teilen, beispielsweise als Blumenkohl, Kartoffeln, geschnittene Möhren und so weiter, sowie Komposite, welche bespielsweise als Pizza oder zusammengesetzte, auf einem Teller angeordnete Speisen, vorliegen können.
Lebensmittel unregelmäßiger Struktur werden in ihrer Morphologie zwar durch eine Verarbeitung zumindest mitbestimmt, wie beispielsweise Käse oder Backwaren, wobei jedoch durch die Verarbeitung keine exakte Vorherbestimmbarkeit der äußeren Beschaffenheit gegeben ist. In jedem Fall handelt es sich um Lebensmittelobjekte, welche anhand ihrer eigenen Beschaffenheit und nicht mittels einer Verpackung erkannt werden müssen.

Die Vorrichtung weist eine Bilderfassungseinheit, eine Auswertungseinheit, eine Dateneingabeeinheit sowie eine Datenausgabeeinheit auf.
Die Auswertungseinheit ist hierbei über Verbindungen mit der Bilderfassungseinheit, der Dateneingabeeinheit und der Datenausgabeeinheit verbunden, wobei die Verbindungen, je nach Anwendungsfall, sowohl drahtgebunden als auch drahtlos ausgebildet sein können.

Die Bilderfassungseinheit ist beispielsweise als Bildkamera ausgebildet, wobei es sich bei der Bildkamera je nach Anwendungsfall um eine Farbwert- oder eine Grauwert-Kamera handeln kann.
Alternativ kann die Bilderfassungseinheit auch in anderen Varianten bildgebender Vorrichtungen, beispielsweise als Tiefenkamera oder in Form eines Computertomografen, vorliegen.
Ebenso ist es erfindungsgemäß möglich, die Bilderfassungseinheit durch mehrere, verschiedene bildgebende Vorrichtungen zu bilden. In diesem Zusammenhang ist es insbesondere möglich, dass die Bilderfassungseinheit beispielsweise durch eine Bildkamera und eine Tiefenkamera gebildet wird.

Mittels der Bilderfassungseinheit ist das Lebensmittelobjekt in Form optischer Daten erfassbar; das bedeutet, dass hierbei, beispielsweise bei Anwendung einer Bildkamera, diskrete Bildpunkte des Lebensmittelobjekt erfasst werden.

Bei einer kombinierten Ausbildung der Bilderfassungseinheit aus Bildkamera und Tiefenkamera werden in diesem Zusammenhang durch die Bildkamera eine Abbildung des Lebensmittelobjekts mit diskreten Bildpunkten und zusätzlich durch die Tiefenkamera Tiefenwerte des Lebensmittelobjekts bereitgestellt, wobei die zusätzliche Einbeziehung der Tiefenwerte hierbei den besonderen Vorteil bietet, dass hierdurch insbesondere eine besonders einfache Abgrenzung des Lebensmittelobjekts gegenüber einer, das Lebensmittelobjekt aufnehmenden, Transportvorrichtung, beispielsweise einem Förderband, bereitstellbar ist. Darüber hinaus können durch die bereitgestellten Tiefenwerte charakteristische Formen des Lebensmittelobjekts, beispielsweise die Bauchhöhle bei einer Schlachttierkörperhälfte, erfasst werden.

Die optischen Daten sind erfindungsgemäß durch die Bilderfassungseinheit an die Auswertungseinheit übertragbar bereitstellbar, wobei die Übertragung der optischen Daten an die Auswertungseinheit über die entsprechende Verbindung erfolgt.

Aus den übertragenen optischen Daten sind mittels der Auswertungseinheit Merkmalswerte des Lebensmittelobjekts extrahierbar.
Bei derartigen Merkmalswerten handelt es sich beispielsweise um Farbwerte, Histogrammmerkmale aus verschiedenen Farbkanälen oder Kanten- und Tiefenmerkmale des Lebensmittelobjekts, welche anhand der optischen Daten, zumindest ausreichend exakt, identifizierbar sind.

Die extrahierten Merkmalswerte des Lebensmittelobjekts sind ferner mittels der Auswertungseinheit zu einem Merkmalswerttupel des Lebensmittelobjekts zusammenfassbar.

Hierbei ist es erfindungsgemäß ebenfalls möglich, dass das Merkmalswerttupel des Lebensmittelobjekts um weitere Datenwerte, wie beispielsweise ein, mittels zusätzlicher Wiegeeinrichtung, vorher oder parallel ermitteltes Gewicht des Lebensmittelobjekts, ergänzt wird.

Des Weiteren sind die gebildeten Merkmalswerttupel des Lebensmittelobjekts automatisch einem Merkmalswerttupelbereich zuordenbar, wobei die Zuordnung des Merkmalswerttupels des Lebensmittelobjekts zu dem Merkmalsbereich auf Basis eines mathematischen Algorithmus, nachfolgend als Klassifikationsalgorithmus bezeichnet, erfolgt.
Ein erfindungsgemäßer Merkmalswerttupelbereich wird hierbei vorausgehend mittels eines weiteren mathematischen Algorithmus, nachfolgend als Bereichsfestlegungsalgorithmus bezeichnet, basierend auf einer repräsentativen Anzahl von Merkmalswerttupeln gebildet.
Mittels des Bereichsfestlegungsalgoritmus und dem oder der zugrundeliegenden Merkmalswerttupel werden somit erfindungsgemäß die Grenzflächen des Merkmalswerttupelbereichs gegenüber weiteren Merkmalswerttupelbereichen, definiert, wobei jeder Merkmalswerttupelbereich einer Klasse entspricht.

Der Merkmalswerttupelbereich kann hierbei in einem n-dimensionalen Merkmalsraum vorliegen, in welchen das Merkmalswerttupel des Lebensmittelobjekts, als Merkmalswertevektor oder als Merkmalswertepunkt, eingetragen ist, sodass das Merkmalswerttupel des Lebensmittelobjekts in dem Merkmalswerttupelbereich und in dem n-dimensionalen Merkmalsraum einen eindeutigen Vektor oder einen eindeutigen Punkt repräsentiert.

Sind in einem Merkmalswerttupelbereich ausreichend viele Merkmalswerttupel eingetragen, so bilden sich innerhalb des Merkmalswerttupelbereichs vorzugsweise Verdichtungen von eingetragenen Merkmalswerttupeln aus.

Dem Merkmalswerttupelbereich ist eine Klasse zuordenbar. Die zugeordnete Klasse bezieht sich in diesem Zusammenhang beispielsweise auf eine, benutzerdefiniert vorgegebene Einteilung unterschiedlicher Lebensmittelobjekte anhand deren Beschaffenheit, wobei jedes zu klassifizierende Lebensmittelobjekte einer diskreten Klasse zuordenbar ist.
Die Zuordnung der Klasse zu dem Merkmalswerttupelbereich ist erfindungsgemäß mittels der Dateneingabeeinheit durchführbar.
Dabei wird die Klasse durch einen Benutzer der Vorrichtung, beispielsweise als konkrete Klassenbezeichnung oder als Artikelnummer, über die Dateneingabeeinheit eingegeben.

Die erfindungsgemäße Vorrichtung zeichnet sich dadurch aus, dass mittels der Auswertungseinheit eine Einteilung des Merkmalswerttupelbereichs in einen Kernbereich und in einen Randbereich bereitstellbar ist.
Der Kernbereich ist erfindungsgemäß so definiert, dass in diesem eine höhere Wahrscheinlichkeit einer korrekten automatischen Zuordnung des Merkmalswerttupels eines Lebensmittelobjekts gegeben ist als in dem Randbereich.

Der Kernbereich kann auch derart verstanden werden, dass in diesem eine höhere Dichte von Merkmalswerttupeln vorliegt, als dies in dem Randbereich der Fall ist, beziehungsweise dass der Abstand von Merkmalswerttupeln zu Merkmalswerttupeln anderer Merkmalswerttupelbereiche relativ groß ist.

Die Einteilung des Merkmalswerttupelbereichs in den Kern- und den Randbereich ist erfindungsgemäß mittels eines Konfidenzschwellenwertes festlegbar.

Der Konfidenzschwellenwert stellt einen festgelegten Wert eines Konfidenzmaßes dar, wobei das Konfidenzmaß ein Maß für die Vertrauenswürdigkeit des automatischen Zuordnungsergebnisses darstellt. Das Konfidenzmaß wird durch einen mathematischen Algorithmus, nachfolgend auch als Konfidenzmaßalgorithmus bezeichnet, gebildet. Ein geringeres Konfidenzmaß drückt eine geringere Wahrscheinlichkeit der Richtigkeit einer automatischen Zuordnung aus und umgekehrt. Einem geringeren Konfidenzmaß entspricht mit anderen Worten eine geringere Erkennungsrate und umgekehrt.

Der Einbezug des bereitgestellten Konfidenzmaßes und des daraus ableitbaren Konfidenzschwellenwertes dient vorliegend insbesondere dazu, fehlerhafte automatische Zuordnungen durch die Vorrichtung soweit als nötig zu vermeiden und gleichzeitig eine möglichst große Effektivität der Vorrichtung dadurch zu erhalten, dass so wenige Zuordnungen wie möglich durch einen Benutzer der Vorrichtung korrigiert oder manuell vorgenommen werden müssen.

Der Konfidenzschwellenwert gibt dabei einen konkreten Wert des Konfidenzmaßes, also des Maßes für die Sicherheit einer korrekten automatischen Zuordnung des Merkmalswerttupels des Lebensmittelobjekts zu dem entsprechenden Merkmalswerttupelbereich, an. Ein geringerer Konfidenzschwellenwert bewirkt eine geringere Wahrscheinlichkeit der Richtigkeit einer automatischen Zuordnung und umgekehrt.
Mit zunehmendem Konfidenzschwellenwert wird die Sicherheit einer potentiell korrekten Zuordnung des Merkmalswerttupels des Lebensmittelobjekts zu dem entsprechenden Merkmalswerttupelbereich höher.

Der Konfidenzschwellenwert zur Einteilung des Merkmalswerttupelbereichs ist erfindungsgemäß mittels der Dateneingabeeinheit benutzergesteuert einstellbar.

Die Einstellung des Konfidenzschwellenwertes durch den Benutzer kann dabei beispielsweise über einen grafischen Schieberegler oder durch Eingabe eines konkreten Zahlenwertes erfolgen.
Da es sich bei dem Konfidenzschwellenwert um einen abstrakten mathematischen Wert handelt, welcher sich für den Benutzer unter Umständen nicht erschließt, erfolgt die Einstellung des Konfidenzschwellenwertes in der Praxis vorzugsweise implizit, zum Beispiel anhand einer grafischen Darstellung einer Funktion einer Erkennungsrate über einer Kernbereichszuordnungsrate, wobei der Konfidenzschwellenwert über einen Arbeitspunkt auf der Funktionskurve gewählt wird.

Erfindungsgemäß sind mittels der Datenausgabeeinheit die Erkennungsrate sowie die Kernbereichszuordnungsrate als abhängige Größen des Konfidenzmaßes und des Konfidenzschwellenwertes ausgebbar.

Die Erkennungsrate gibt dabei die Wahrscheinlichkeit einer Menge korrekter Zuordnungen von Merkmalswerttupeln zu dem entsprechenden Merkmalswerttupelbereich, bezogen auf eine Gesamtmenge durchgeführter Zuordnungen an. Umgekehrt betrachtet stellt die Erkennungsrate ein Maß einer Fehlerhäufigkeit der automatischen Zuordnungen dar; bei einer geringen Erkennungsrate liegt eine hohe Fehlerhäufigkeit vor, bei einer hohen Erkennungsrate liegt dagegen eine geringe Fehlerhäufigkeit vor.

Demgegenüber gibt die Kernbereichszuordnungsrate die Größe des Kernbereichs bezogen auf den Merkmalswerttupelbereich an, womit bei einer hohen Kernbereichzuordnungsrate die Anzahl der Lebensmittelobjekt, die dem Kernbereich zugeordnet werden im Verhältnis zu der Gesamtmenge der Zuordnungen hoch ist.

Dabei werden Zuordnungen im Kernbereich vorzugsweise durch automatische Zuordnungen von Merkmalswerttupeln durch die Vorrichtung vorgenommen. Die Vorrichtung arbeitet in diesem Fall im Kernbereich also autark.
Sowohl im Bezug auf die Erkennungsrate als auch im Bezug auf die Kernbereichszuordnungsrate gilt, dass es sich jeweils um Prognoseaussagen handelt, welche für zukünftig durchzuführende Zuordnungen bestimmt und festgelegt werden sollen.

Dabei stehen die Erkennungsrate und die Kernbereichszuordnungsrate zueinander in einem inversen Abhängigkeitsverhältnis. Die Abhängigkeit ist derart ausgebildet, dass bei einem geringen Konfidenzmaß eine geringe Erkennungsrate und demgegenüber eine hohe Kernbereichzuordnungsrate vorliegen und umgekehrt.

Wird der Konfidenzschwellenwert also auf ein solches niedriges Konfidenzmaß eingestellt, wäre das Ergebnis der durch die Vorrichtung vorgenommenen automatischen Zuordnung von Merkmalswerttupeln zu einem Merkmalswerttupelbereich als weniger vertrauenswürdig einzustufen, da eine höhere Wahrscheinlichkeit einer höheren Anzahl inkorrekter Zuordnungen von Merkmalswerttupeln zu dem Merkmalswerttupelbereich vorliegt.
Im Gegenzug liegt jedoch eine größere Anzahl von Kernbereichszuordnungen bzw. eine höhere Kernbereichszuordnungsrate vor, sodass eine hohe Anzahl automatischer Zuordnungen beziehungsweise eine geringere Anzahl manueller Zuordnungen durchführbar ist. Dies bedeutet, dass ein verringerter Zeit- und Kostenaufwand für manuelle Zuordnungen, insbesondere ein geringerer Kostenaufwand für Personal, vorliegt.

Setzt man alternativ den Konfidenzschwellenwert beispielsweise verhältnismäßig hoch an, so würde dies zu einer hohen Erkennungsrate und einer niedrigen Kernbereichszuordnungsrate führen. Dies bedeutet, dass die Anzahl der durch die Vorrichtung automatisch vorgenommenen Zuordnungen sinkt, dafür aber das Ergebnis der automatisch vorgenommenen Zuordnungen als vertrauenswürdiger einzustufen ist. Im Gegenzug steigt hierbei jedoch die Anzahl der Randbereichszuordnungen welche vorzugsweise manuell, beispielsweise durch den Benutzer, durchzuführen sind, wodurch sich der Aufwand und somit beispielsweise die Personalkosten erhöhen würden.

Die Vorrichtung ermöglicht es somit auf besonders vorteilhafte Art und Weise, dass der Benutzer der Vorrichtung das zu erwartende Zuordnungsergebnis mittels der Anpassung des Konfidenzschwellenwertes nach seinen persönlichen Vorgaben anpassen und nach den konkreten Bedingungen optimieren kann. Die Optimierung stellt eine Abwägung zwischen dem Grad der Qualität der automatisch vorgenommenen Zuordnungen und dem Aufwand für manuelle Zuordnungen oder Kontrollen dar. Ein Vorteil der Erfindung besteht darin, dass die Vorrichtung gemäß der jeweiligen unternehmerischen Wertentscheidung optimiert und betrieben werden kann.

Dabei kann die Ausgabe der Erkennungsrate und der Kernbereichszuordnungsrate beispielsweise anhand eines Graphen erfolgen, bei welchem die Erkennungsrate als abhängige Größe der Kernbereichszuordnungsrate und umgekehrt dargestellt sind.
Ergänzend ist es ebenfalls möglich, Erkennungs- und Kernbereichszuordnungsrate derart auszugeben, dass der jeweilige Konfidenzschwellenwert als eingebbare oder wählbare Größe zugeordnet ist.

Im Bezug auf die erfindungsgemäß bereitstellbare Einteilung des Merkmalswerttupelbereichs in Kern- und Randbereich erfolgt die automatische Zuordnung des Merkmalswerttupels des Lebensmittelobjekts erfindungsgemäß getrennt nach Kern- und Randbereich. Das Merkmalswerttupel des Lebensmittelobjekts wird somit durch die Vorrichtung entweder dem Kernbereich oder dem Randbereich zugeordnet. Durch eine veränderte Einstellung des Konfidenzschwellenwerts kann sich auch eine Veränderung der Zuordnung zu dem Kernbereich oder dem Randbereich ergeben.

Das Ergebnis der Zuordnung des Merkmalswerttupels des Lebensmittelobjekts zu dem Merkmalswerttupelbereich ist durch die erfindungsgemäße Vorrichtung als Zuordnungsergebnis ausgebbar.
Die Ausgabe des Zuordnungsergebnisses erfolgt besonders vorteilhaft getrennt als Zuordnung zu dem Kernbereich oder als Zuordnung zu dem Randbereich. Die Zuordnung zu dem Kernbereich wird in diesem Zusammenhang als Kernbereichszuordnung und die Zuordnung zu dem Randbereich als Randbereichszuordnung bezeichnet.
Speziell im Falle einer Kernbereichszuordnung kann die Ausgabe des Zuordnungsergebnisses unter Angabe der zugeordneten Klasse, beispielsweise als Grafik mit einem hinterlegten Bild der jeweiligen Klasse oder mit einer konkreten Artikelbezeichnung oder Artikelnummer erfolgen.

In einer vorzugsweisen Variante ist die Vorrichtung ferner dazu in der Lage, mittels der Bilderfassungseinheit eine eventuell bereits vorhandene Kennzeichnung des Lebensmittelobjekts, beispielsweise durch einen Strich- oder Barcode zu erfassen.
In diesem Fall wäre es erfindungsgemäß möglich, das erfasste Lebensmittelobjekt direkt einer entsprechenden Klasse zuzuordnen, ohne dass die oben beschriebene Kern- oder Randbereichsbetrachtung durchgeführt werden muss.

Hierbei wird es vorteilhaft ermöglicht, dass mittels der vorhandenen Kennzeichnung visuell nicht oder schlecht unterscheidbare Lebensmittelobjekte klassifizierbar gemacht werden, ohne dass diese logistisch separat behandelt werden müssen.

Ferner kann es in diesem Zusammenhang ermöglicht werden, anhand der oben aufgeführten Zuordnung des Merkmalswerttupels des Lebensmittelobjekts, eine Überprüfung durchzuführen, ob die bereits vorhandene Kennzeichnung des Lebensmittelobjekts, mittels des Strich- oder Barcodes, korrekt ist oder ob eine fehlerhafte Kennzeichnung vorliegt.
Umgekehrt kann auf diese Weise eine Überprüfung der zuverlässigen Funktion der Vorrichtung anhand der automatisch vorgenommenen Zuordnungsergebnisse durchgeführt werden.

Des Weiteren ist die Vorrichtung vorzugsweise dazu in der Lage, in Abhängigkeit des Zuordnungsergebnisses, weitere technische Vorrichtungen, beispielsweise Sortieranlagen, anzusteuern, um so das Lebensmittelobjekt zum Beispiel zur Überprüfung an einen manuellen Zuordnungspunkt oder an eine Verpackungsstation weiterzuleiten.

Als weiterer Vorteil kann erfindungsgemäß eine klassenspezifische Festlegung des Konfidenzschwellenwertes vorgenommen werden.
In diesem Zusammenhang kann beispielsweise für eine Klasse ein spezifischer Konfidenzschwellenwert hinterlegt werden, welcher dann nur für diese eine Klasse angewendet wird. Andere Klassen würden dann vorzugsweise mit einem anderen Konfidenzschwellenwert versehen werden.
Auf diese Weise wird es ermöglicht, dass die Erkennungs- und die Kernbereichszuordnungsrate für jede Klasse unterschiedlich gewählt werden können.

Eine vorteilhafte Weiterbildung der erfindungsgemäßen Vorrichtung sieht vor, dass durch diese, mittels Kernbereichszuordnung, eine automatische Klassifikation bereitstellbar ist.
In Abhängigkeit des jeweils gewählten Konfidenzschwellenwertes wird hierbei das Merkmalswerttupel des Lebensmittelobjekts, sofern es dem Kernbereich des Merkmalswerttupelbereichs zugeordnet wurde, und somit das Lebensmittelobjekt, durch die Vorrichtung automatisch klassifiziert, ohne dass eine manuelle Zuordnung durchgeführt werden muss.

Die hier aufgeführte Weiterbildung hat insbesondere den Vorteil, dass Merkmalswerttupel, welche zu dem Kernbereich des jeweiligen Merkmalswerttupelbereichs zugeordnet werden können, durch die Vorrichtung automatisch und, je nach gewähltem Konfidenzschwellenwert mit einer prognostizierten Erkennungsrate, klassifiziert werden können. Ein zusätzlicher Aufwand durch eine notwendige manuelle Überprüfung des Zuordnungsergebnisses entfällt somit.

Darüber hinaus sieht eine vorteilhafte Weiterbildung der Erfindung vor, dass mittels Randbereichszuordnung eine Klassifikation als nicht oder alternativ als nicht zuverlässig durchführbar ausgebbar ist.
Wenn eine Randbereichszuordnung vorliegt, erfolgt durch die Vorrichtung eine zusätzliche Datenausgabe, vorzugsweise als eine Informationsausgabe an einen Benutzer, deren Inhalt voreinstellbar ist. Beispielsweise kann die Information ausgegeben werden, dass eine Klassifikation des Lebensmittelobjekts nicht durchführbar ist. In diesem Fall würde dann eine manuelle Klassifikation durchgeführt werden. In einer anderen Variante wird die Information ausgegeben, dass die Klassifikation des Lebensmittelobjekts möglicherweise fehlerhaft sein könnte. In dieser Variante kann das Klassifikationsergebnis durch den Benutzer nochmals kontrolliert werden. Die zusätzliche Datenausgabe kann alternativ oder kumulativ auch als Steuerbefehl für den weiteren Prozess erfolgen. So ist es beispielsweise möglich, eine automatische Klassifikation auszuschließen und eine manuelle Zuordnung zu erzwingen. Als ein anderes Beispiel kann die automatische Klassifikation trotzdem zugelassen aber das Lebensmittelobjekt auf ein se-_ parates Transportband zur Nachkontrolle geleitet werden.

Diese Weiterbildung bietet insbesondere den technologischen Vorteil, dass Zuordnungen mit einer geringeren Erkennungsrate und damit Zuordnungsvorgänge mit einer höheren Wahrscheinlichkeit einer fehlerhaften Zuordnung des Lebensmittelobjekts durch den Benutzer der Vorrichtung, rechtzeitig erkannt und verhindert werden kann oder dass eine manuelle Zuordnung erzwungen wird, was zwar mit einem zusätzlichen Aufwand verbunden ist, aber im Ergebnis Fehler weitgehend ausschließt.

In einer vorteilhaften Variante kann, je nach Benutzerfestlegung, klassenspezifisch eine Randbereichszuordnung unterschiedlich ausgegeben werden. Beispielsweise wird hierbei in einer ersten Klasse eine Klassifikation in dem Randbereich als nicht durchführbar und in einer zweiten Klasse eine Klassifikation zwar durchgeführt, aber als nicht zuverlässig durchführbar ausgegeben.

Eine weitere vorteilhafte Ausbildung der Erfindung sieht vor, dass die automatische Zuordnung des Merkmalswerttupels des Lebensmittelobjekts zu dem Merkmalswerttupelbereich benutzergesteuert überwachbar und korrigierbar ist.

Durch den Benutzer kann in diesem Zusammenhang beispielsweise der gesamte Zuordnungsprozess überwacht werden, wobei der Benutzer ein Zuordnungsergebnis sowohl bei einer Ausgabe als Kernbereichszuordnung als auch bei einer Ausgabe als Randbereichszuordnung überwachen und optional korrigieren kann.

In diesem Zusammenhang ist es beispielsweise auch möglich, dass die Vorrichtung in einem sogenannten Überwachungs- oder Trainingsmodus betreibbar ist. Der Überwachungs- oder Trainingsmodus beschreibt hierbei einen Vorgang, bei welchem beispielsweise die automatische Zuordnung durch die Vorrichtung parallel zu einer manuellen Zuordnung durch einen Benutzer durchgeführt wird. Die Vorrichtung führt in diesem Fall zwar als Echtbetrieb eine automatische Zuornung eines Lebensmittelobjekts durch; das Zuordnungsergebnis ist jedoch durch den Benutzer in Echtzeit überwachbar, sodass dieser bei einer erkannten Fehlklassifikation oder einer, als nicht durchführbar angegebenen, Klassifikation in den Zuordnungsprozess eingreifen um so eine korrekte Zuordnung des Merkmalswerttupels des Lebensmittelobjekts zu dem Merkmalswerttupelbereich und somit eine korrekte Klassifikation des Lebensmittelobjekts durchzuführen.
Das Ergebnis der manuellen Klassifikation kann dann wiederum an die Vorrichtung zurückgeführt und diese somit trainiert werden.

Im Rahmen eines solchen Überwachungs- oder Trainingsmodus ist es erfindungsgemäß ferner möglich, dass die Vorrichtung mittels der Bilderfassungseinheit lediglich die optischen Daten des Lebensmittelobjekts erfasst und hieraus das Merkmalswerttupel des Lebensmittelobjekts bildet. Die anschließende Zuordnung des Merkmalswerttupels des Lebensmittelobjekts zu dem entsprechenden Merkmalswerttupelbereich wird dann durch den Benutzer durchgeführt und das korrekte Zuordnungsergebnis anschließend an die Vorrichtung zurückgeführt. Es erfolgt somit im übertragenen Sinne ein Anlernen der Vorrichtung.

Des Weiteren wird es durch die hier aufgeführte Ausbildung besonders vorteilhaft ermöglicht, dass durch den Benutzer der Vorrichtung eine nachträgliche Überwachung und gegebenenfalls Korrektur der Zuordnungsergebnisse, beispielsweise in einem Überprüfungs- oder Review-Modus, durchführbar ist. Je nach Ergebnis der Zuordnungen können fehlerhafte Zuordnungen dann nachgelagert korrigiert und die korrigierten Zuordnungsergebnisse an die Vorrichtung zurückgeführt werden.

Ein solcher Review-Modus kann beispielsweise am Ende jeder Schicht vorgesehen werden, wobei die Zuordnungsergebnisse durch den Benutzer entweder nur stichprobenartig oder komplett überprüft und wenn notwendig korrigiert werden können.

In jedem Fall werden korrigierte Zuordnungsergebnisse, nach deren Rückführung an die Vorrichtung, mittels des Bereichsfestlegungsalgorithmus in die Definition des jeweiligen Merkmalswerttupelbereichs einbezogen und so die Vorrichtung weiter trainiert.

Ferner kann vorzugsweise am Ende eines Review-Modus eine Übersicht über die fehlerhaften oder als nicht automatisch durchführbar deklarierten Zuordnungen ausgegeben werden, sodass der Benutzer der Vorrichtung entscheiden kann, ob das Gesamtergebnis der automatischen Zuordnungen und somit die Effektivität der Vorrichtung ausreichend ist oder ob gegebenenfalls noch zusätzliche Verfeinerungen durch ein zusätzliches Training notwendig sind.

In einer besonders vorteilhaften Weiterbildung der Erfindung sind, bei Vorhandensein mehrerer Zuordnungsergebnisse unterschiedlicher Lebensmittelobjekte, die Zuordnungsergebnisse geordnet, in Abhängigkeit eines Konfidenzmaßes, bereitstellbar.

Die Bereitstellung erfolgt hierbei beispielsweise anhand einer Tabelle, in welcher die Zuordnungsergebnisse nach aufsteigendem Konfidenzmaß geordnet aufgeführt werden.

Auf diese Weise wird es, insbesondere bei einer Überprüfung der Zuordnungsergebnisse im Rahmen eines Review-Modus besonders vorteilhaft ermöglicht, dass bei der Überprüfung zuerst die Zuordnungsergebnisse mit einem geringen Konfidenzmaß und danach erst die Zuordnungsergebnisse mit einem hohen Konfidenzmaß aufgeführt werden.
Dem Benutzer der Vorrichtung wird es somit im Rahmen des Review-Modus ermöglicht, zuerst die weniger vertrauenswürdigen Zuordnungsergebnisse und anschließend die höher vertrauenswürdigen Zuordnungsergebnisse zu begutachten, sodass beispielsweise der Review-Modus nach Erreichen einer festgelegten Grenze der Vertrauenswürdigkeit in die Zuordnungsergebnisse abgebrochen werden kann, da ab diesem Zeitpunkt mit einer hohen Wahrscheinlichkeit nur noch richtige Zuordnungsergebnisse vorliegen.

Die hier aufgezeigte Weiterbildung bietet somit insbesondere den technologischen Vorteil, dass der benötigte Zusatzaufwand und etwaige zusätzliche Personalkosten für eine Überprüfung der Zuordnungsergebnisse reduziert werden können.

Als weiterer Vorteil kann zuverlässig ermittelt werden, bei welchem Konfidenzmaß welche Erkennungsrate vorliegt um hieraus abzuleiten, mit welchem Konfidenzschwellenwert die Vorrichtung optimiert betrieben werden kann.

Die erfassten optischen Daten sowie das jeweils zugehörige Zuordnungsergebnis sind in einer weiteren vorteilhaften Variante der Erfindung archivierbar.

Mittels der Archivierung wird es hierbei besonders vorteilhaft ermöglicht, dass die optischen Daten mit den zugehörigen Zuordnungsergebnissen und insbesondere die jeweilige Einstellung des Konfidenzschwellenwertes nachträglich noch einmal nachvollzogen werden können. Zusätzlich können weitere Daten, wie beispielsweise der eingestellte Konfidenzschwellenwert, hierzu zugeordnet mit archiviert werden.
Auf diese Weise kann der Betreiber der Vorrichtung belegen, dass er die Vorrichtung mit angemessenen Konfidenzschwellenwerteinstellungen betrieben hat und sich beispielsweise vor Schadensersatzforderungen wegen angeblich riskanten Anlagenbetriebes schützen.

Die Archivierung der optischen Daten nebst den zugehörigen Zuordnungsergebnissen erfolgt beispielsweise in einem Datenspeicher der Auswertungseinheit selbst oder in einem externen Datenspeicher.

Eine besonders vorteilhafte Weiterbildung der erfindungsgemäßen Vorrichtung sieht ferner vor, dass mittels der Bilderfassungseinheit das Lebensmittelobjekt als optische Daten auf einem Transportsystem erfassbar und das jeweilige Zuordnungsergebnis in Echtzeit bereitstellbar ist.
Mittels des Zuordnungsergebnisses sind anschließend externe Einheiten steuerbar.

Bei den externen Einheiten handelt es sich beispielsweise um Sortiereinheiten, welche, basierend auf dem Zuordnungsergebnis und mittels der Steuerung durch die Auswertungseinheit, eine Sortierung des Lebensmittelobjekts, insbesondere nach Klassen oder beispielsweise auch nach automatisch klassifiziert oder nach nicht, beziehungsweise nicht zuverlässig klassifiziert, durchführen. Darüber hinaus kann es sich bei den externen Einheiten beispielsweise auch um Verpackungseinheiten handeln, welche das Lebensmittelobjekt nach der Zuordnung, entsprechend dem Zuordnungsergebnis, passend verpacken.

Ein erfindungsgemäßes Verfahren zur Klassifikation eines Lebensmittelobjekts gewachsener oder unregelmäßiger Struktur wird mittels einer Vorrichtung, aufweisend eine Bilderfassungseinheit, eine Auswertungseinheit, eine Dateneingabeeinheit und eine Datenausgabeeinheit, durchgeführt, wobei die Auswertungseinheit mit der Bilderfassungseinheit, der Dateneingabeeinheit und der Datenausgabeeinheit verbunden ist.

Dabei weist das Verfahren erfindungsgemäß folgende Verfahrensschritte auf:
a) Erfassen des Lebensmittelobjekts als optische Daten durch die Bilderfassungseinheit,
b) Übertragen der optischen Daten an die Auswertungseinheit,
c) Extrahieren von Merkmalswerten des Lebensmittelobjekts aus den optischen Daten durch die Auswertungseinheit,
d) Zusammenfassen der Merkmalswerte des Lebensmittelobjekts zu einem Merkmalswerttupel des Lebensmittelobjekts durch die Auswertungseinheit,
e) Zuordnen einer Klasse zu einem Merkmalswerttupelbereich durch die Dateneingabeeinheit, wobei der Merkmalswerttupelbereich aus einem oder mehreren Merkmalswerttupeln gebildet wird,
f) Einteilung des Merkmalswerttupelbereichs in einen Kernbereich und einen Randbereich mittels Eingabe eines Konfidenzschwellenwertes, wobei eine Größe des Kernbereichs durch ein benutzergesteuertes Einstellen des Konfidenzschwellenwertes mittels der Dateneingabeeinheit bestimmt wird,
g) Ausgeben einer Erkennungsrate und einer Kernbereichszuordnungsrate als abhängige Größen des Konfidenzschwellenwertes durch die Datenausgabeeinheit,
h) automatisches Zuordnen des Merkmalswerttupels des Lebensmittelobjekts zu dem Merkmalswerttupelbereich, wobei die Zuordnung entweder zu dem Kernbereich oder zu dem Randbereich erfolgt,
i) Ausgeben des Ergebnisses der Zuordnung des Merkmalswerttupels des Lebensmittelobjekts zu dem Merkmalswerttupelbereich als Zuordnungsergebnis, unter Angabe der zugeordneten Klasse, durch die Datenausgabeeinheit, wobei die Ausgabe des Zuordnungsergebnisses mit einer Zuordnung zu dem Kernbereich, als Kernbereichszuordnung, oder mit einer Zuordnung zu dem Randbereich, als Randbereichszuordnung, erfolgt.

In dem ersten Verfahrensschritt a) erfolgt das Erfassen des Lebensmittelobjekts als optische Daten durch die Bilderfassungseinheit.
Hierbei ist die Bilderfassungseinheit beispielsweise als Bildkamera, vorliegend als Farbwert- oder Grauwert-Kamera, ausgebildet.
Mittels der Bilderfassungseinheit wird das Lebensmittelobjekt in Form optischer Daten erfasst; dies bedeutet, dass hierbei, beispielsweise bei Anwendung einer Bildkamera, diskrete Bildpunkte des Lebensmittelobjekts erfasst werden.
Die erfassten optischen Daten des Lebensmittelobjekts werden darüber hinaus durch die Bilderfassungseinheit übertragbar bereitgestellt.

In dem sich anschließenden Verfahrensschritt b) erfolgt das Übertragen der optischen Daten von der Bilderfassungseinheit an die Auswertungseinheit mittels der entsprechenden Verbindung.

Die Auswertungseinheit nimmt in Verfahrensschritt c) eine Extraktion von Merkmalswerten des Lebensmittelobjekts aus den übertragenen optischen Daten vor. Bei den extrahierten Merkmalswerten handelt es sich beispielsweise um Farbwerte, Histogrammmerkmale aus verschiedenen Farbkanälen oder Kanten- und Tiefenmerkmale des Lebensmittelobjekts, Abmessungen und so weiter, welche anhand der optischen Daten, zumindest ausreichend exakt, identifizierbar sind.

In Verfahrensschritt d) nimmt die Auswertungseinheit anschließend die Zusammenfassung der Merkmalswerte des Lebensmittelobjekts zu einem Merkmalswerttupel des Lebensmittelobjekts vor.

Im Zuge dieser Zusammenfassung ist es erfindungsgemäß möglich, dass das Merkmalswerttupel des Lebensmittelobjekts um weitere Datenwerte, wie beispielsweise ein, mittels zusätzlicher Wiegeeinrichtung, vorher oder parallel ermitteltes Gewicht des Lebensmittelobjekts, ergänzt wird.

Des Weiteren wird in dem Verfahrensschritt e) mittels der Dateneingabeeinheit, einem Merkmalswerttupelbereich eine Klasse zugeordnet. Dabei wird die Klasse durch einen Benutzer der Vorrichtung, beispielsweise als konkrete Klassenbezeichnung oder als Artikelnummer, über die Dateneingabeeinheit eingegeben. Dem Wesen nach handelt es sich um die Vergabe einer Bezeichnung für den betreffenden Merkmalswerttupelbereich.

Der Merkmalswerttupelbereich wurde hierbei mittels eines mathematischen Algorithmus, nachfolgend als Bereichsfestlegungsalgorithmus bezeichnet, aus einer repräsentativen Anzahl von Merkmalswerttupeln gebildet, wobei mittels des Bereichsfestlegungsalgoritmus und dem oder der jeweils zugrundeliegenden Merkmalswerttupel die Grenzflächen des Merkmalswerttupelbereichs, insbesondere auch gegenüber weiteren Merkmalswerttupelbereichen, definiert worden sind.
In diesem Zusammenhang kann die repräsentative Anzahl vorliegend sowohl durch ein repräsentatives Merkmalswerttupel oder durch eine Mehrzahl repräsentativer Merkmalswerttupel gebildet werden.

Der Merkmalswerttupelbereich kann hierbei beispielsweise als n-dimensionaler Merkmalsraum vorliegen, in welchen das Merkmalswerttupel des Lebensmittelobjekts, zum Beispiel als Merkmalswertevektor oder als Merkmalswertepunkt, eingetragen wird, sodass das Merkmalswerttupel des Lebensmittelobjekts in dem Merkmalswerttupelbereich beispielsweise einen eindeutigen Vektor oder einen eindeutigen Punkt repräsentiert.

Ferner erfolgt in Verfahrensschritt f) die Einteilung des Merkmalswerttupelbereichs in Kernbereich und Randbereich.
Die Einteilung wird hierbei mittels Eingabe des Konfidenzschwellenwertes bereitgestellt, wobei durch die benutzergesteuerte Einstellung des Konfidenzschwellenwertes die Größe des Kernbereichs bestimmt wird.
Der Kernbereich ist hierbei erfindungsgemäß so definiert, dass in diesem eine höhere Wahrscheinlichkeit einer korrekten automatischen Zuordnung des Merkmalswerttupels des Lebensmittelobjekts gegeben ist als in dem Randbereich.

Die Grundlage für den Konfidenzschwellenwert bildet erfindungsgemäß ein vorher festgelegtes Konfidenzmaß, welches ein Maß für die Vertrauenswürdigkeit des automatischen Zuordnungsergebnisses darstellt.
Das Konfidenzmaß wird in diesem Zusammenhang ebenfalls durch einen mathematischen Algorithmus, nachfolgend auch als Konfidenzmaßalgorithmus bezeichnet, gebildet.
Der Konfidenzschwellenwert stellt erfindungsgemäß einen konkreten, benutzergesteuert einstellbaren Wert des Konfidenzmaßes dar.

Durch die Datenausgabeeinheit werden in Verfahrensschritt g) eine Erkennungsrate und eine Kernbereichzuordnungsrate, als abhängige Größen des Konfidenzschwellenwertes ausgegeben.
Die Erkennungsrate gibt dabei eine Menge korrekter Zuordnungen von Merkmalswerttupeln zu dem entsprechenden Merkmalswerttupelbereich, bezogen auf eine Gesamtmenge durchgeführter Zuordnungen an.
Demgegenüber gibt die Kernbereichszuordnungsrate die Größe des Kernbereichs bezogen auf den Merkmalswerttupelbereich an, wobei bei einer hohen Kernbereichzuordnungsrate eine hohe Anzahl automatischer Zuordnungen von Merkmalswerttupeln durch die Vorrichtung vorgenommen wird.

Die Ausgabe der Erkennungsrate und der Kernbereichszuordnungsrate kann vorliegend beispielsweise anhand eines Graphen erfolgen, in welchem der jeweilige Konfidenzschwellenwert als eingebbare oder wählbare Größe, beispielsweise als grafischer Schieber oder als positionierbarer Datenpunkt, hinterlegt ist.

In dem Verfahrensschritt h) erfolgt schließlich eine automatische Zuordnung des Merkmalswerttupels des Lebensmittelobjekts zu dem Merkmalswerttupelbereich. Die Zuordnung wird hierbei erfindungsgemäß getrennt nach Kern- und Randbereich vorgenommen, sodass das Merkmalswerttupel des Lebensmittelobjekts entweder dem Kernbereich oder dem Randbereich zugeordnet wird.
Die Zuordnung basiert auf einem mathematischen Algorithmus, nachfolgend als Klassifikationsalgorithmus bezeichnet. Sie stellt den Klassifikationsvorgang im engeren Sinne dar.

Abschließend wird in Verfahrensschritt i) das Ergebnis der Zuordnung des Merkmalswerttupels des Lebensmittelobjekts zu dem Merkmalswerttupelbereich als Zuordnungsergebnis ausgegeben.
Die Ausgabe des Zuordnungsergebnisses erfolgt dabei getrennt nach einer Zuordnung zu dem Kernbereich, vorliegend als Kernbereichzuordnung bezeichnet, oder einer Zuordnung zu dem Randbereich, vorliegend als Randbereichszuordnung bezeichnet. Als getrennte Ausgabe kann es auch verstanden werden, dass dem Zuordnungsergebnis eine Zusatzinformation darüber beigegeben wird, ob es sich um eine Kernbereichszuordnung oder eine Randbereichszuordnung handelt.
Speziell im Falle einer Kernbereichszuordnung erfolgt die Ausgabe des Zuordnungsergebnisses unter Angabe der zugeordneten Klasse, beispielsweise als Grafik mit einem hinterlegten Bild der jeweiligen Klasse oder mit einer konkreten Artikelbezeichnung oder Artikelnummer.

Eine Reihenfolge der Verfahrensschritte ist für das erfindungsgemäße Verfahren lediglich insofern vorgegeben, als innerhalb der Verfahrensschrittgruppe a) bis d) die Verfahrensschritte jeweils nacheinander ausgeführt werden müssen und dass die Verfahrensschritte h) und nachfolgend i) die Durchführung der anderen Verfahrensschritte voraussetzt. Im Übrigen werden keine Reihenfolgenfestlegungen getroffen.

In einer vorteilhaften Weiterbildung des Verfahrens wird bei einer Kernbereichszuordnung des Merkmalswerttupels des Lebensmittelobjekts durch die Auswertungseinheit der Vorrichtung eine automatische Klassifikation des Lebensmittelobjekts vorgenommen.

Darüber hinaus sieht eine weitere vorteilhafte Variante des Verfahrens vor, dass durch die Auswertungseinheit der Vorrichtung bei einer Randbereichszuordnung des Merkmalswerttupels des Lebensmittelobjekts eine Klassifikation als nicht oder nicht zuverlässig durchführbar ausgegeben wird.
In diesem Fall wird eine entsprechende Information von der Auswertungseinheit an die Datenausgabeeinheit übersendet und durch die Datenausgabeeinheit visualisiert, sodass ein Benutzer der Vorrichtung gegebenenfalls die Klassifikation überprüft oder darüber informiert wird, dass eine Klassifikation in diesem Fall durch die Vorrichtung nicht vorgenommen wurde.

In einer besonders vorteilhaften Weiterbildung des Verfahrens wird nach dem Verfahrensschritt i) ein zusätzlicher Verfahrensschritt j) ausgeführt.

In dem Verfahrensschritt j) erfolgt erfindungsgemäß eine benutzergesteuerte Überwachung und optional eine Korrektur der automatischen Zuordnung des Merkmalswerttupels des Lebensmittelobjekts zu dem Merkmalswerttupelbereich.

Hierbei wird durch den Benutzer der Vorrichtung beispielsweise der gesamte Zuordnungsprozess in Echtzeit oder im Rahmen einer Nachbearbeitung, zum Beispiel während eines Überprüfungs- oder Review-Modus, überwacht werden, wobei der Benutzer ein Zuordnungsergebnis insbesondere, sowohl bei einer Ausgabe des Zuordnungsergebnisses als Kernbereichszuordnung als auch bei einer Ausgabe des Zuordnungsergebnisses als Randbereichszuordnung, korrigieren kann.
Ein korrigiertes Ergebnis kann hierbei durch den Benutzer mittels der Dateneingabeeinheit an die Auswertungseinheit der Vorrichtung zurückgeführt und diese so trainiert werden.

Ferner sieht eine weitere vorteilhafte Variante des Verfahrens vor, dass, bei einem Vorhandensein mehrerer Zuordnungsergebnisse unterschiedlicher Lebensmittelobjekt, die Zuordnungsergebnisse geordnet in Abhängigkeit von einem Konfidenzmaß ausgegeben werden.
Die Ausgabe der Zuordnungsergebnisse erfolgt hierbei beispielsweise anhand einer Tabelle, in welcher die Zuordnungsergebnisse nach aufsteigendem Konfidenzmaß geordnet aufgeführt werden. Der Vorteil dieser Weiterbildung des Verfahrens besteht vor allem darin, dass zuverlässige Informationen über die erzielbaren Erkennungsraten und Kernbereichszuordnungsraten in Abhängigkeit des Konfidenzmaßes gewonnen werden können, auf deren Basis die Optimierung der Verfahrensdurchführung mittels Konfidenzschwellenwerteinstellung erfolgen kann.

In einer vorteilhaften Weiterbildung des Verfahrens ist ein zusätzlicher Verfahrensschritt k) durchführbar.

Der Verfahrensschritt k) wird vorzugsweise nach dem Verfahrensschritt i) oder j) durchgeführt und sieht erfindungsgemäß ein Archivieren der optischen Daten und des zugehörigen Zuordnungsergebnisses vor.

Das Archivieren der optischen Daten und des zugehörigen Zuordnungsergebnisses erfolgt beispielsweise in einer Speichereinheit der Auswertungseinheit oder in einer externen Speichereinheit, welche mit der Auswertungseinheit verbunden ist.

Die Erfindung wird nachfolgend als Ausführungsbeispiel einer Vorrichtung zur Klassifikation von Schlachttierkörperobjekten, anhand von
Fig. 1 Prinzipdarstellung mit Bildkamera
Fig. 2 Prinzipdarstellung mit Bild- und Tiefenkamera
Fig. 3 grafische Darstellung Kernbereichs-/Erkennungsrate in Abhängigkeit des Konfidenzmaßes
Fig. 4 grafische Darstellung Erkennungsrate in Abhängigkeit der Kernbereichszuordnungsrate
näher erläutert.

Eine erfindungsgemäße Vorrichtung zur Klassifikation eines Lebensmittelobjekts 1, vorliegend als Schlachttierkörperobjekt, ist im hier gezeigten Ausführungsbeispiel als Bestandteil eines sogenannten Identifikationspunktes ausgeführt.
Der Identifikationspunkt stellt in diesem Zusammenhang eine Station dar, an welcher objektbezogene Daten des Lebensmittelobjekts 1 ermittelt und für eine Weiterverarbeitung des Lebensmittelobjekts 1 bereitgestellt werden.

Dementsprechend sind dem Identifikationspunkt ferner Steuerelemente zur Ansteuerung weiterer, das Lebensmittelobjekt 1 verteilender oder weiterverarbeitender, Einrichtungen zugeordnet.

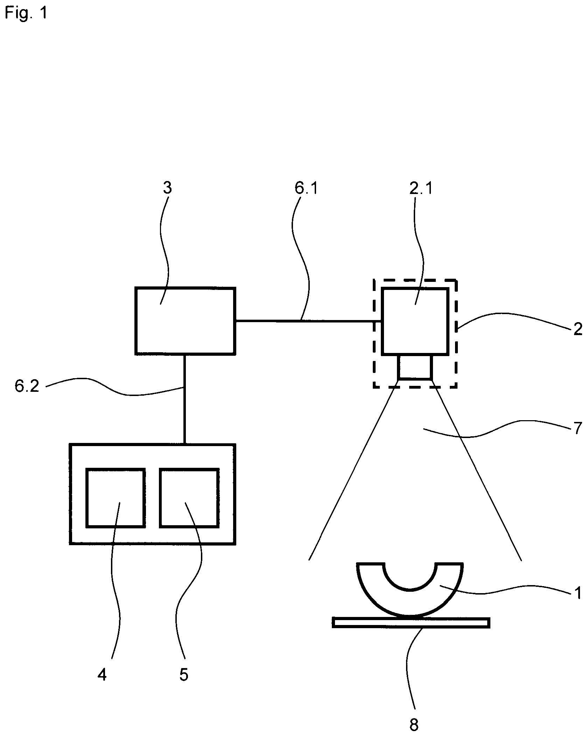

Die Vorrichtung zur Klassifikation des Lebensmittelobjekts 1 weist erfindungsgemäß eine Bilderfassungseinheit 2, eine Auswertungseinheit 3, eine Dateneingabeeinheit 4 sowie eine Datenausgabeeinheit 5 auf.
Die Auswertungseinheit 3 ist dabei mit der Bilderfassungseinheit 2, der Dateneingabeeinheit 4 und der Datenausgabeeinheit 5 verbunden.
Des Weiteren wird die Bilderfassungseinheit 2 gemäß Fig. 1 durch eine Bildkamera 2.1, vorliegend als Farbkamera ausgeführt, mit einem Bildkameraerfassungsbereich 7 gebildet.
Die Dateneingabeeinheit 4 sowie die Datenausgabeeinheit 5 werden ferner durch jeweils ein Display gebildet und sind in einer Einheit zusammengefasst.

Mittels der Bilderfassungseinheit 2 ist das Lebensmittelobjekt 1 als optische Daten erfassbar.
Bei der vorliegenden Ausführung gemäß Fig. 1 stellen die optischen Daten diskrete Bildpunkte des Lebensmittelobjekts 1 dar, welche von der Bildkamera erfasst werden.
Die optischen Daten werden darüber hinaus durch die Bilderfassungseinheit 2 an die Auswertungseinheit 3 übertragbar bereitgestellt.

Mittels der Auswertungseinheit 3 sind erfindungsgemäß Merkmalswerte aus den optischen Daten extrahierbar.
Bei den extrahierbaren Merkmalswerten handelt es sich beispielsweise um Farbwerte des Lebensmittelobjekts 1, Histogrammmerkmale aus verschiedenen Farbkanälen oder um Kantenmerkmale des Lebensmittelobjekts 1.

Die Auswertungseinheit 3 ist darüber hinaus erfindungsgemäß dazu in der Lage, die extrahierten Merkmalswerte des Lebensmittelobjekts 1 zu einem Merkmalswerttupel des Lebensmittelobjekts 1 zusammenzufassen.

Im vorliegenden Ausführungsbeispiel erfolgt die Zusammenfassung der Merkmalswerte anhand einer Vektorbildung, wobei ein Merkmalswerttupel einen Merkmalswertevektor darstellt und somit das Lebensmittelobjekt 1 durch diesen konkreten Merkmalswertevektor repräsentiert wird.

Erfindungsgemäß wird mittels der Dateneingabeeinheit 4 einem Merkmalswerttupelbereich eine Klasse, vorliegend eine Schlachttierkörperklasse, zugeordnet. Ein solcher Merkmalswerttupelbereich ist erfindungsgemäß mittels eines mathematischen Algorithmus, nachfolgend als Bereichsfestlegungsalgorithmus bezeichnet, basierend auf mindestens einem Merkmalswerttupel gebildet worden. Dies bedeutet, dass ein Merkmalswerttupelbereich durch den Bereichsfestlegungsalgorithmus beispielsweise auf Basis eines einzigen repräsentativen Merkmalswerttupels und somit auf Basis eines einzigen Lebensmittelobjekts 1 oder basierend auf einer Mehrzahl repräsentativer Merkmalswerttupel und somit basierend auf mehreren Lebensmittelobjekten definiert worden ist.
Durch den zugrunde liegenden Bereichsfestlegungsalgorithmus werden in diesem Zusammenhang die Grenzflächen des jeweiligen Merkmalswerttupelbereichs gegenüber den Grenzflächen weiterer Merkmalswerttupelbereiche definiert.
Anders formuliert wird durch den Bereichsfestlegungsalgorithmus mit den Grenzflächen eine "Hülle" festgelegt, die den Merkmalswerttupelbereich um das mindestens eine Merkmalswerttupel definiert und begrenzt.

Im vorliegenden Ausführungsbeispiel liegt der Merkmalswerttupelbereich in einem n-dimensionalen Merkmalsraum vor, wobei das Merkmalswerttupel des Lebensmittelobjekts 1 als Merkmalswertevektor in diesen Merkmalsraum eingetragen ist.
Das Lebensmittelobjekt 1 wird somit in dem Merkmalsraum durch den eingetragenen Merkmalswertevektor repräsentiert.
Es liegen darüber hinaus in dem Merkmalsraum weitere Merkmalswerttupelbereiche vor, welche mittels des Bereichsfestlegungsalgorithmus anhand repräsentativer Anzahlen von Merkmalswerttupeln weiterer Lebensmittelobjekt anderer Klassen definiert werden.

Die Klasse wird vorliegend durch einen Benutzer der Vorrichtung als Artikelnummer mit zugehöriger Klassenbezeichnung über die Dateneingabeeinheit 4 eingegeben.
Erfindungsgemäß spielt es hierbei keine Rolle, ob die Zuordnung der Klasse zu dem Merkmalswerttupelbereich vor der Zuordnung des jeweiligen Merkmalswerttupels, oder erst im Nachgang an die Zuordnung des Merkmalswerttupels, vorgenommen wird. Für eine praktische Bedienung wird es jedoch regelmäßig sinnvoll sein, den Merkmalswerttupelbereich mittels Vergabe einer Klassenbezeichnung bzw. Artikelnummer alsbald benennbar zu machen.

Ein wesentliches Merkmal besteht darin, dass mittels der Auswertungseinheit 3 eine Einteilung des Merkmalswerttupelbereichs in einen Kernbereich und in einen Randbereich bereitstellbar ist.
Der Kernbereich stellt dabei den Abschnitt des Merkmalswerttupelbereichs dar, in welchem, bei einem Vorhandensein mehrerer Merkmalswerttupel gattungsgleicher Lebensmittelobjekt, eine höhere Dichte von Merkmalswerttupeln als in dem Randbereich vorliegt, beziehungsweise in welchem der Abstand der zugeordneten Merkmalswerttupel zu weiteren Merkmalswerttupeln anderer Merkmalswerttupelbereiche relativ groß ist.

Die Einteilung des Merkmalswerttupelbereichs durch die Auswertungseinheit 3 erfolgt erfindungsgemäß anhand eines Konfidenzschwellenwertes.
Dieser Konfidenzschwellenwert stellt einen festgelegten Wert eines sogenannten Konfidenzmaßes dar, wobei das Konfidenzmaß ein Maß für die Vertrauenswürdigkeit eines, durch die Vorrichtung vorgenommenen, automatischen Zuordnungsergebnisses darstellt.
Das Konfidenzmaß wird im vorliegenden Ausführungsbeispiel durch einen weiteren mathematischen Algorithmus, nachfolgend als Konfidenzmaßalgorithmus bezeichnet, gebildet.

Demzufolge gibt der Konfidenzschwellenwert vorliegend einen konkreten Wert für das Maß der Sicherheit einer korrekten automatischen Zuordnung des Merkmalswerttupels des Lebensmittelobjekts 1 zu dem entsprechenden Merkmalswerttupelbereich an.

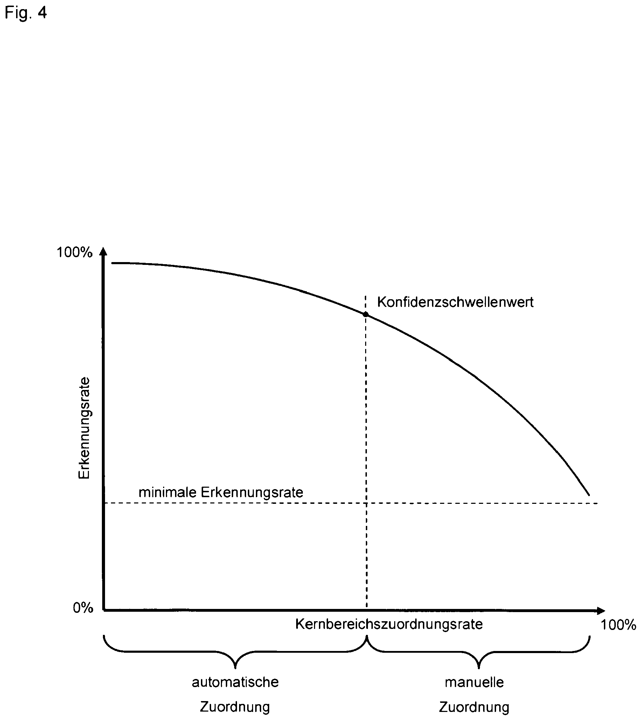

Erfindungsgemäß ist der Konfidenzschwellenwert und somit das Konfidenzmaß benutzergesteuert mittels der Dateneingabeeinheit 4 einstellbar.
Dabei erfolgt die Einstellung des Konfidenzschwellenwertes über die Dateneingabeeinheit 4 vorliegend, wie in Fig. 4 dargestellt, mittelbar anhand eines wählbaren Arbeitspunktes einer grafisch dargestellten Funktion einer Erkennungsrate in Abhängigkeit von einer Kernbereichszuordnungsrate.
Erfindungsgemäß gibt hierbei die Erkennungsrate die Wahrscheinlichkeit einer Menge korrekter Zuordnungen von Merkmalswerttupeln zu dem entsprechenden Merkmalswerttupelbereich, bezogen auf eine Gesamtmenge durchgeführter Zuordnungen an.
Demgegenüber stellt die Kernbereichszuordnungsrate ein Maß für die Größe des Kernbereichs, bezogen auf den Merkmalswerttupelbereich dar.

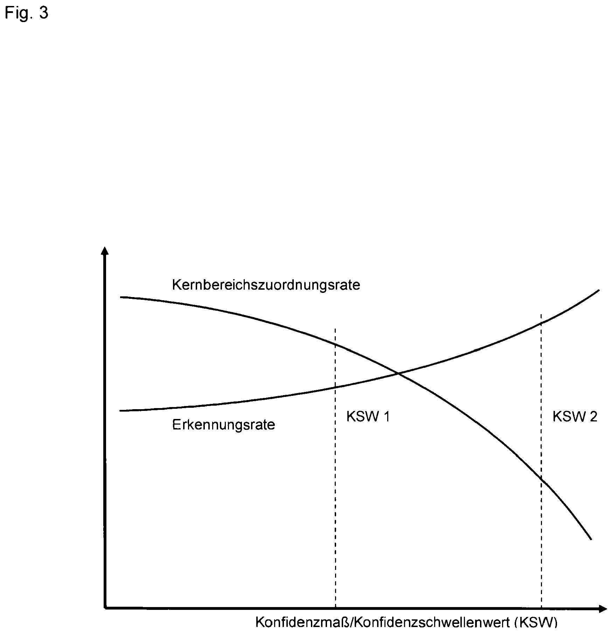

Wie in Fig. 3 dargestellt, stehen die Erkennungsrate und die Kernbereichszuordnungsrate erfindungsgemäß in einem inversen Verhältnis zueinander; dies bedeutet, dass bei einem niedrigen Konfidenzmaß eine geringe Erkennungsrate und gleichzeitig eine hohe Kernbereichszuordnungsrate vorliegen.
Werden der Konfidenzschwellenwert und somit das Konfidenzmaß niedrig gewählt, so ist das Ergebnis der, durch die erfindungsgemäße Vorrichtung vorgenommenen automatischen Zuordnung des Merkmalswerttupels zu einem Merkmalswerttupelbereich als weniger vertrauenswürdig anzusehen, da aufgrund der groß festgelegten Kernbereichs mit daraus folgender großer Kernbereichszuordnungsrate die Wahrscheinlichkeit inkorrekter Zuordnungen vergleichsweise hoch ist. Gleichzeitig würde in diesem Fall durch die Vorrichtung eine große Menge automatischer Zuordnungen vorgenommen werden.

Setzt man im Gegensatz dazu einen hohen Konfidenzschwellenwert und somit ein hohes Konfidenzmaß an, so führt dies zu einer hohen Erkennungsrate und zu einer niedrigen Kernbereichszuordnungsrate.
Bei einer Konfiguration, bei der Kernbereichszuordnungen als automatische Zuordnungen ausgeführt werden, würde anders ausgedrückt in diesem Fall die Menge der durch die Vorrichtung automatisch vorgenommenen Zuordnungen sinken, jedoch gleichzeitig die Vertrauenswürdigkeit in die automatisch vorgenommenen Zuordnungen steigen, da nur eine entsprechend kleine Kernbereichszuordnungsrate vorliegt.
Hieraus ergibt sich der technologische Vorteil der Erfindung, dass ein Besitzer oder ein Benutzer der Vorrichtung über den Konfidenzschwellenwert festlegen kann, mit welcher potentiellen Sicherheit korrekter automatischer Zuordnungen und somit wie autark die Vorrichtung arbeiten soll. In der praktischen Handhabung kann die Festlegung erkennungsratengeführt erfolgen, wobei dann die Kernbereichszuordnungsrate als abhängige Größe ausgegeben wird, oder kernbereichszuordnungsratengeführt erfolgen, wobei dann die Erkennungsrate als abhängige Größe ausgegeben wird.

Ferner ist die Auswertungseinheit 3 erfindungsgemäß in der Lage, das Merkmalswerttupel des Lebensmittelobjekts 1 dem Merkmalswerttupelbereich automatisch zuzuordnen. Die Zuordnung erfolgt vorliegend getrennt nach Kern- oder Randbereich, sodass das Merkmalswerttupel des Lebensmittelobjekts 1 durch die Auswertungseinheit 3 entweder dem Kernbereich oder dem Randbereich zugeordnet wird.
Die Zuordnung des Merkmalswerttupels des Lebensmittelobjekts 1 zu dem Merkmalswerttupelbereich stellt erfindungsgemäß den Abschluss des Klassifikationsvorgangs dar.

Das Ergebnis einer, durch die Vorrichtung vorgenommenen Zuordnung des Merkmalswerttupels des Lebensmittelobjekts 1 zu dem jeweiligen Merkmalswerttupelbereich, wird erfindungsgemäß durch die Datenausgabeeinheit 5 als Zuordnungsergebnis ausgegeben.

Als besonderer Vorteil der Erfindung erfolgt die Ausgabe des Zuordnungsergebnisses entweder als Zuordnung zu dem Kernbereich oder als Zuordnung zu dem Randbereich, wobei in einer besonders vorteilhaften Ausführungsvariante der Erfindung eine Zuordnung zu dem Kernbereich, als Kernbereichszuordnung, durch die Vorrichtung automatisch durchgeführt wird und wobei eine Zuordnung zu dem Randbereich, als Randbereichszuordnung, durch die Vorrichtung als nicht oder alternativ nicht zuverlässig durchführbar ausgegeben wird.
Eine Randbereichszuordnung führt in diesem Fall dazu, dass das jeweilige Lebensmittelobjekt 1 noch einmal, vorzugsweise manuell, überprüft und dementsprechend manuell klassifiziert wird.

In diesem Zusammenhang zeigt Fig. 4 die Funktion der Erkennungsrate über der Kernbereichszuordnungsrate in Abhängigkeit des gewählten Konfidenzschwellenwertes.
Wie Fig. 4 zu entnehmen ist, liegt die Kernbereichszuordnungsrate je nach gewähltem Konfidenzschwellenwert zwischen 0% und 100%, wobei bei einer hohen Kernbereichszuordnungsrate zwar eine große Anzahl automatischer Zuordnungen vorliegt, jedoch nur eine geringe Erkennungsrate vorliegt und somit die Zuordnungsergebnisse nur ein geringes Konfidenzmaß aufweisen.
Gleichzeitig wird aus Fig. 4 ersichtlich, dass bei sinkender Kernbereichszuordnungsrate weniger automatische Zuordnungen durch die Vorrichtung durchgeführt werden, die Erkennungsrate jedoch vergleichsweise hoch ist.
Somit weisen die wenigen durchgeführten automatischen Zuordnungen ein hohes Konfidenzmaß auf.
Zusätzlich zeigt Fig. 4, dass die Erkennungsrate nicht auf 0% abfallen wird, da auch bei einer Kernbereichszuordnungsrate von 100%, wenn auch teilweise lediglich zufällig, korrekte automatische Zuordnung durch die Vorrichtung vorgenommen werden.

Die erfindungsgemäße Vorrichtung erlaubt besonders vorteilhaft unterschiedliche Betriebsmodi, welche nachfolgend verfahrensartig veranschaulicht werden.

Ein erster möglicher Betriebsmodus stellt einen Trainingsmodus dar, welcher eine Erstinbetriebnahme und ein Anlernen der Vorrichtung vorsieht.
Der Trainingsmodus bezieht sich dabei vorliegend auf eine bevorzugte Ausführungsform der Erfindung, bei welcher ein sogenanntes Training mit Annotation erfolgt was bedeutet, dass einem erfindungsgemäß definierten Merkmalswerttupelbereich stets eine entsprechende Kennzeichnung, vorliegend eine Klasse, insbesondere durch Eingabe durch den Benutzer der Vorrichtung, zugeordnet ist. Die Zuordnung der Klasse durch den Benutzer kann wahlweise vor oder parallel zur Definition des jeweiligen Merkmalswerttupelbereichs oder nach dessen Definition durchgeführt werden.

Dem Trainingsmodus liegt ferner zugrunde, dass die Vorrichtung bis zu diesem Zeitpunkt noch keine automatische Zuordnung eines Merkmalswerttupels eines Lebensmittelobjekts 1 zu einem Merkmalswerttupelbereich vorgenommen hat und ein solcher Merkmalswerttupelbereich bis zu diesem Zeitpunkt noch nicht vorliegt.

Nach der Erstinbetriebnahme der Vorrichtung erfolgt in dem Trainingsmodus ein Erfassen des zu klassifizierenden Lebensmittelobjekts 1 durch die Bilderfassungseinheit 2 als optische Daten.
Die optischen Daten werden anschließend von der Bilderfassungseinheit 2 an die Auswertungseinheit 3 übertragen.

Nach dem Empfang der optischen Daten extrahiert die Auswertungseinheit 3 aus diesen konkrete Merkmalswerte des Lebensmittelobjekts 1, wie beispielsweise Farb- oder Kantenwerte.
Die extrahierten Merkmalswerte des Lebensmittelobjekts 1 werden im Anschluss, wie oben bereits beschrieben, durch die Auswertungseinheit 3 zu einem Merk-malswerttupel des Lebensmittelobjekts 1 zusammengefasst.

Auf Basis einer repräsentativen Anzahl von Merkmalswerttupeln von Lebensmittelobjekten erfolgen ferner mittels des Bereichsfestlegungsalgorithmus die Bildung eines zugehörigen Merkmalswerttupelbereichs in dem n-dimensionalen Merkmalsraum mit Definition der Grenzflächen des Merkmalswerttupelbereichs gegenüber weiteren Merkmalswerttupelbereichen sowie mittels des Konfidenzmaßalgorithmus die Grundlage für die Unterteilung des Merkmalswerttupelbereichs in einen Kern- und einen Randbereich.
Der gebildete Merkmalswerttupelbereich wird in dem vorliegenden Ausführungsbeispiel durch den Benutzer der Vorrichtung mit einer konkreten Klasse, in diesem Fall einer Schlachttierkörperobjektklasse, versehen.
Nachfolgend wird in diesem Zusammenhang nur die konkrete, dem Merkmalswerttupelbereich des Lebensmittelobjekts 1 zugeordnete Klasse betrachtet, wobei zugrunde gelegt werden kann, dass auch alle, in dem Merkmalsraum definierten Merkmalswerttupelbereiche mit jeweils einer Klasse versehen sind.

Im weiteren Verlauf des Trainingsmodus werden weitere Lebensmittelobjekte durch die Bilderfassungseinheit 2 erfasst und aus diesen, auf die oben beschriebene Art und Weise, jeweils ein Merkmalswerttupel gebildet.
Je nach Ausbildung der neu gebildeten Merkmalswerttupel werden diese entweder dem bereits vorhandenen Merkmalswerttupelbereich zugeordnet oder eine Zuordnung wird als nicht durchführbar ausgegeben.
Bei gattungsmäßig abweichenden Lebensmittelobjekten ist es der Vorrichtung auch möglich, aus den nicht zuordenbaren Merkmalswerttupeln jeweils neue Merkmalswerttupelbereiche zu bilden. Aufgrund der Komplexität der vorliegend zu klassifizierenden Schlachttierkörperobjekte ist eine solche eigenständige Neudefinition von Merkmalswerttupelbereichen jedoch im Ausführungsbeispiel nicht vorgesehen.

Der Trainingsmodus wird vorliegend parallel zu einer manuellen Klassifikation des Lebensmittelobjekts 1 durchgeführt, wobei die manuelle Klassifikation durch einen Benutzer der Vorrichtung ausgeführt wird.
In diesem Zusammenhang wird das Lebensmittelobjekt 1 durch die Vorrichtung zwar klassifiziert oder eine Klassifikation als nicht durchführbar ausgegeben während jedoch der eigentliche, reale Klassifikationsvorgang durch den Benutzer der Vorrichtung manuell, durch Zuordnung des Merkmalswerttupels des Lebensmittelobjekts 1 zu dem korrekten Merkmalswerttupelbereich, durchgeführt wird.

Der Trainingsmodus bietet den besonderen Vorteil, dass nach der automatischen Zuordnung durch die Vorrichtung das Zuordnungsergebnis mit dem der manuellen Zuordnung verglichen und so die Korrektheit des automatischen Zuordnungsergebnisses überprüft werden kann.
Auf diese Weise kann, bei einer als nicht durchführbar ausgegebenen oder fehlerhaften Zuordnung das korrekte Zuordnungsergebnis durch den Benutzer, in Echtzeit oder nachgelagert, aus der manuellen Zuordnung an die Vorrichtung übermittelt werden, sodass diese eine Anpassung des Merkmalswerttupelbereichs aufgrund des korrigierten Ergebnisses durchführen und somit trainiert werden kann.

Während des Trainingsmodus kann die manuelle Zuordnung mit anschließender Rückübermittlung der Daten an die Vorrichtung entweder zeitgleich mit der automatischen Zuordnung oder am Ende eines länger dauernden Zuordnungszyklus als nachgelagertes Training durchgeführt werden.

Im Ergebnis des Trainingsmodus liegt in der Vorrichtung ein ausreichend großer Datensatz von Zuordnungen vor, um eine anwenderspezifische Vertrauenswürdigkeit in die Zuordnungsergebnisse gewährleisten zu können.

Einen zweiten möglichen Betriebsmodus bildet vorliegend ein Testmodus, in welchem die Vorrichtung in einer benutzerüberwachten Arbeitsweise zwar eine automatische Zuordnung des Merkmalswerttupels des Lebensmittelobjekts 1 vornimmt, jedoch aufgrund der automatischen Zuordnungen noch keine, das Lebensmittelobjekt 1 weiterverarbeitenden, Einrichtungen angesteuert werden.
In dem Testmodus besteht vorliegend die Möglichkeit, dass der Benutzer das automatische Zuordnungsergebnis in Echtzeit überprüfen und gegebenenfalls korrigierend eingreifen kann.

Erst nach erfolgter Bestätigung des Zuordnungsergebnisses durch den Benutzer werden die Zuordnungsergebnisse für weiterführende Anwendungen verwendet.

Die Bestätigung durch den Benutzer kann beispielsweise auf die Art erfolgen, dass die Vorrichtung über die Datenausgabeeinheit 4 das aktuelle Zuordnungsergebnis anzeigt und bei Nichtreaktion des Benutzers das Zuordnungsergebnis als korrekt verzeichnet wird.
Liegt demgegenüber ein inkorrektes Zuordnungsergebnis vor oder wurde die Zuordnung durch die Vorrichtung als nicht oder nicht zuverlässig durchführbar ausgegeben, so hat der Benutzer die Möglichkeit, innerhalb einer festgelegten Zeitspanne, das Zuordnungsergebnis mittels Eingabe an der Dateneingabeeinheit 3 vorzunehmen oder zu korrigieren.

Den dritten Betriebsmodus bildet der Autarkbetriebmodus, in welchem die Vorrichtung im Echtbetrieb selbstständig eine automatische Zuordnung des Merkmalswerttupels des Lebensmittelobjekts 1 zu dem entsprechenden Merkmalswerttupel-bereich und somit autark eine Klassifikation des Lebensmittelobjekts 1 vornimmt, beziehungsweise eine Klassifikation als nicht oder nicht zuverlässig durchführbar ausgibt, sowie nachgelagerte Einrichtungen zur Weiterverarbeitung des Lebens-mittelobjekts 1 ansteuert.

Bei einer automatisch vorgenommenen Klassifikation des Lebensmittelobjekts 1 kann die Vorrichtung hierbei beispielsweise ein Transportsystem 10 derart steuern, dass das Lebensmittelobjekt 1 zu einer entsprechenden Zerlege- oder Verpackungseinrichtung befördert wird.
Bei einer nicht oder nicht korrekt durchführbaren Zuordnung kann alternativ das Transportsystem 10 durch die Vorrichtung so angesteuert werden, dass das Lebensmittelobjekt 1 zu einer weiteren Überprüfung einem manuellen Identifikationspunkt zugeführt wird. Bei dem Autarkbetriebsmodus handelt es sich um den bevorzugten Regelbetrieb.

Darüber hinaus kann die Vorrichtung als weiteren Betriebszustand in einem Überprüfungsmodus, auch als Review-Modus bezeichnet, betrieben werden. Der Review-Modus stellt vorliegend eine besonders vorteilhafte Zusatzfunktion der Vorrichtung dar und wird nach einem bereits durchgeführten Zuordnungszyklus, entweder im Anschluss an den Trainingsmodus oder im Anschluss an den Autarkbetriebsmodus, beispielsweise am Ende einer Schicht, durchgeführt, wobei in dem Review-Modus durch den Benutzer der Vorrichtung eine Überprüfung der vorgenommenen Zuordnungsergebnisse vorgenommen wird.

In dem Review-Modus werden dem Benutzer sowohl die Zuordnungsergebnisse als auch parallel die bildlichen Darstellungen der zugeordneten Lebensmittelobjekte, beispielsweise als Bildergalerie oder in einer Zuordnungstabelle, dargestellt.
In einer Ausführungsform werden die Zuordnungsergebnisse dabei nach aufsteigendem Konfidenzmaß geordnet ausgegeben.
Die geordnete Ausgabe der Zuordnungsergebnisse bietet dabei insbesondere den Vorteil, dass zuerst die Zuordnungsergebnisse mit einem geringen Konfidenzmaß und somit die weniger vertrauenswürdigen Zuordnungsergebnisse ausgegeben werden, bei denen eine höhere Wahrscheinlichkeit an Fehlzuordnungen, auch als Fehlklassifikationen zu bezeichnen, oder anders ausgedrückt eine geringere Erkennungsrate vorliegt.
Nach Überprüfung der Menge an Zuordnungsergebnissen mit geringem Konfidenzmaß folgen dann nur noch Zuordnungsergebnisse mit einem hohen Konfidenzmaß, sodass davon ausgegangen werden kann, dass diese Zuordnungsergebnisse potentiell richtig sind.
Somit müssen innerhalb des Review-Modus nicht alle Zuordnungsergebnisse noch einmal überprüft werden, sodass der personelle und der zeitliche Aufwand zur Überprüfung besonders gering gehalten werden kann.

Im Ergebnis des Review-Modus können ferner die Anzahl Fehlklassifikationen und als nicht durchführbar ausgegebener Klassifikationen durch die Datenausgabeeinheit 5 ausgegeben werden, sodass der Benutzer der Vorrichtung entscheiden kann, ob die Vorrichtung eine ausreichend hohe Wahrscheinlichkeit richtiger Zuordnungen gewährleisten kann oder ob noch weitere Trainingsdaten notwendig sind. Dem Benutzer stehen damit zugleich die Informationen zur Verfügung, bei welcher Erkennungsrate welche Kernbereichszuordnungsrate vorliegt und wie die Einstellung entsprechend den Benutzerpräferenzen optimiert werden kann.

Die erfindungsgemäße Vorrichtung bietet in diesem Zusammenhang in einer bevorzugten Variante die Option, dass die erfassten optischen Daten des Lebensmittelobjekts 1 und das zugehörige Zuordnungsergebnis archivierbar sind.
Auf diese Weise wird es vorteilhaft ermöglicht, dass, beispielsweise bei späteren Haftungsfragen, die Zuordnungsergebnisse sowie die Wahrscheinlichkeit korrekter automatischer Zuordnungen in Abhängigkeit des jeweils gewählten Konfidenzschwellenwertes nachvollziehbar sind.

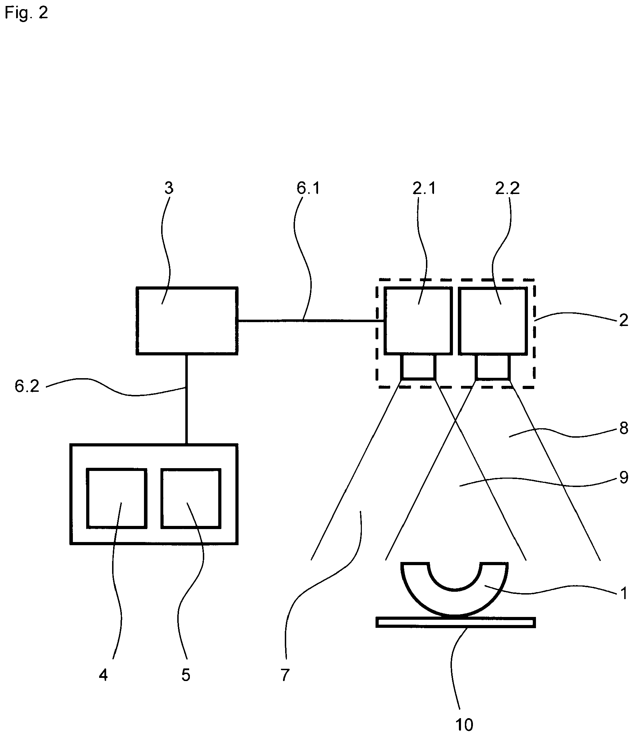

Eine besonders vorteilhafte Weiterbildung der Erfindung gemäß Fig. 2 sieht vor, dass die Bilderfassungseinheit 2 sowohl aus der Bildkamera 2.1 als auch aus einer Tiefenkamera 2.2 mit einem Tiefenkameraerfassungsbereich 8 gebildet wird.

Die Tiefenkamera 2.2 ist erfindungsgemäß so angeordnet, dass sich der Bildkameraerfassungsbereich 7 und der Tiefenkameraerfassungsbereich 8 in einem gemeinsamen Erfassungsbereich 9 überschneiden, wobei in dem gemeinsamen Erfassungsbereich 9 der, für die Klassifikation relevante, Abschnitt des Lebensmittelobjekts 1 erfasst wird.

Die zusätzlich vorgesehene Tiefenkamera 2.2 bietet hierbei insbesondere den technologischen Vorteil, dass neben den Farbwerten auch Tiefenwerte des Lebensmittelobjekts 1, wie sie beispielsweise bei einer Bauchhöhle einer Schlachttierkörperhälfte auftreten, erfassbar sind.
Somit können zusätzliche Merkmalswerte des Lebensmittelobjekts 1 erfasst und darauf aufbauend ein umfangreicheres Merkmalswerttupel des Lebensmittelobjekts 1 bereitgestellt werden.

Eine weitere bevorzugte Ausführungsform der Erfindung sieht, wie in Fig. 1 und 2 dargestellt, vor, dass mittels der Bilderfassungseinheit 2 das Lebensmittelobjekt 1 als optische Daten auf einem Transportsystem 10 erfassbar das jeweilige Zuordnungsergebnis in Echtzeit bereitstellbar ist.

In einer weiteren Ausführungsform sind die Bilderfassungseinheit 2 einerseits und die Dateneingabeeinheit 4 sowie die Datenausgabeeinheit 5 räumlich getrennt zueinander angeordnet. Dies ermöglicht es, den Identifikationspunkt von einem zentralen Leitstand, der sich auch in einer anderen Betriebsstätte befinden kann, zu überwachen und zu steuern. In einer Abwandlung ist die Dateneingabeeinheit 4 sowie die Datenausgabeeinheit 5 gerätetechnisch umschaltbar ausgebildet, so dass eine Überwachung und Steuerung beispielsweise umschaltbar ist zwischen direkt (lokal) am Transportsystem 10 oder an einem zentralen Leitstand (remote) oder mehreren Stellen gleichzeitig.

In einer dazu verwandten Ausführungsform wird der Identifikationspunkt in mehreren parallelen Linien betrieben. Dies bedeutet, dass mehrere Bilderfassungseinheiten 2 jeweils zugehörigen Auswertungseinheiten 3 und Dateneingabeneinheiten 4 und Datenausgabeeinheiten 5 vorhanden sind, so dass mehrere Lebensmittelobjekte parallel erfasst werden können. Dabei sind in dem Ausführungsbeispiel die mehreren Auswertungseinheiten 3, Dateneingabeeinheiten 4 und Dateneingabeeinheiten 5 gerätetechnisch zusammengefasst, so das mit nur einer Bedienperson von einem zentralen Leitstand aus mehrere parallele Erfassungen und Zuordnungen überwachbar sind, wodurch der Personalseinsatz weiter reduziert und die Effektivität erheblich gesteigert werden kann. Dabei wird der besondere Vorteil der Erfindung ausgenutzt, dass nur die Lebensmittelobjekte, die dem Randbereich zugehören, zur visuell-menschlichen Entscheidung über die Zuordnung an die Bedienperson übergeben werden. Über die Einstellung des Konfidenzschwellwertes kann die Anzahl der bei mehreren Linien anfallenden Zuordnungsentscheidungen, die durch die Bedienpersonen zu treffen sind, auf ein arbeitsphysiologisch und betriebswirtschaftlich zweckmäßiges Maß eingestellt werden.

### Verwendete Bezugszeichen

- 1: Lebensmittelobjekt
- 2: Bilderfassungseinheit
- 2.1: Bildkamera
- 2.2: Tiefenkamera
- 3: Auswertungseinheit
- 4: Dateneingabeeinheit
- 5: Datenausgabeeinheit
- 6: Verbindung
- 7: Bildkameraerfassungsbereich
- 8: Tiefenkameraerfassungsbereich
- 9: gemeinsamer Erfassungsbereich Bildkamera/Tiefenkamera
- 10: Transportsystem

## Patentansprüche

1. Vorrichtung zur Klassifikation eines Lebensmittelobjekts (1) gewachsener oder unregelmäßiger Struktur,
aufweisend eine Bilderfassungseinheit (2), eine Auswertungseinheit (3), eine Dateneingabeeinheit (4) und eine Datenausgabeeinheit (5), wobei die Auswertungseinheit (3) mit der Bilderfassungseinheit (2), der Dateneingabeeinheit (3) und der Datenausgabeeinheit (4) verbunden ist, und wobei mittels der Bilderfassungseinheit (2) das Lebensmittelobjekt (1) als optische Daten erfassbar ist und die optischen Daten übertragbar an die Auswertungseinheit (3) bereitstellbar sind, und wobei mittels der Auswertungseinheit (3) aus den optischen Daten Merkmalswerte des Lebensmittelobjekts (1) extrahierbar sind, wobei die Merkmalswerte zu einem Merkmalswerttupel des Lebensmittelobjekts (1) zusammenfassbar sind und wobei das Merkmalswerttupel des Lebensmittelobjekts (1) einem Merkmalswerttupelbereich automatisch zuordenbar ist, wobei der Merkmalswerttupelbereich durch ein oder mehrere Merkmalswerttupel gebildet wird und wobei dem Merkmalswerttupelbereich eine Klasse zuordenbar ist, und wobei mittels der Dateneingabeeinheit (4) die Zuordnung der Klasse zu dem Merkmalswerttupelbereich durchführbar ist,
**dadurch gekennzeichnet,**
**dass** mittels der Auswertungseinheit (3) eine Einteilung des Merkmalswerttupelbereichs in einen Kernbereich und einen Randbereich bereitstellbar ist, wobei in dem Kernbereich eine höhere Wahrscheinlichkeit einer korrekten Zuordnung des Merkmalswerttupels des Lebensmittelobjekts (1) zu dem Merkmalswerttupelbereich gegeben ist als in dem Randbereich,
und **dass** die Einteilung des Merkmalswerttupelbereichs mittels eines Konfidenzschwellenwertes festlegbar ist, wobei durch den Konfidenzschwellenwert eine Größe des Kernbereichs festlegbar ist,
und **dass** der Konfidenzschwellenwert mittels der Dateneingabeeinheit (4) benutzergesteuert einstellbar ist,
und **dass** mittels der Datenausgabeeinheit (5) eine Erkennungsrate und eine Kernbereichszuordnungsrate, als abhängige Größen des Konfidenzschwellenwerts, ausgebbar sind,
und **dass** die automatische Zuordnung des Merkmalswerttupels des Lebensmittelobjekts zu dem Merkmalswerttupelbereich getrennt nach Kernbereich und Randbereich bereitstellbar ist,
und **dass** das Ergebnis der Zuordnung des Merkmalswerttupels des Lebensmittelobjekts zu dem Merkmalswerttupelbereich, unter Angabe der zugeordneten Klasse, als Zuordnungsergebnis, mit einer Zuordnung zu dem Kernbereich, als Kernbereichszuordnung, oder mit einer Zuordnung zu dem Randbereich, als Randbereichszuordnung, ausgebbar ist.

2. Vorrichtung nach Anspruch 1,
**dadurch gekennzeichnet,**
**dass** mittels der Kernbereichszuordnung eine automatische Klassifikation bereitstellbar ist.

3. Vorrichtung nach Anspruch 1 oder 2,
**dadurch gekennzeichnet,**
**dass** mittels der Randbereichszuordnung eine Klassifikation als nicht oder nicht zuverlässig durchführbar ausgebbar ist.

4. Vorrichtung nach einem der vorhergehenden Ansprüche,
**dadurch gekennzeichnet,**
**dass** die automatische Zuordnung des Merkmalswerttupels des Lebensmittelobjekts (1) zu dem Merkmalswerttupelbereich benutzergesteuert überwachbar und korrigierbar ist.

5. Vorrichtung nach einem der vorhergehenden Ansprüche,
**dadurch gekennzeichnet,**
**dass**, bei einem Vorhandensein mehrerer Zuordnungsergebnisse unterschiedlicher Lebensmittelobjekt, eine Ausgabe der Zuordnungsergebnisse geordnet in Abhängigkeit von einem Konfidenzmaß bereitstellbar ist.

6. Vorrichtung nach einem der vorhergehenden Ansprüche,
**dadurch gekennzeichnet,**
**dass** die optischen Daten und das zugehörige Zuordnungsergebnis archivierbar sind.

7. Vorrichtung nach einem der vorhergehenden Ansprüche,
**dadurch gekennzeichnet,**
**dass** mittels der Bilderfassungseinheit (2) das Lebensmittelobjekt (1) als optische Daten auf einem Transportsystem (10) erfassbar ist und dass das Zuordnungsergebnis in Echtzeit bereitstellbar ist und dass mittels des Zuordnungsergebnisses externe Einheiten steuerbar sind.

8. Verfahren zur Klassifikation eines Lebensmittelobjekts (1) gewachsener oder unregelmäßiger Struktur,
mittels einer Bilderfassungseinheit (2), einer Auswertungseinheit (3), einer Dateneingabeeinheit (4) und einer Datenausgabeeinheit (5), wobei die Auswertungseinheit (3) mit der Bilderfassungseinheit (2), der Dateneingabeeinheit (4) und der Datenausgabeeinheit (5) verbunden ist, aufweisend die nachfolgenden Verfahrensschritte:
a) Erfassen des Lebensmittelobjekts 1 als optische Daten durch die Bilderfassungseinheit (2),
b) Übertragen der optischen Daten an die Auswertungseinheit (3),
c) Extrahieren von Merkmalswerten des Lebensmittelobjekts 1 aus den optischen Daten durch die Auswertungseinheit (3),
d) Zusammenfassen der Merkmalswerte des Lebensmittelobjekts 1 zu einem Merkmalswerttupel des Lebensmittelobjekts 1 durch die Auswertungseinheit (3),
e) Zuordnen einer Klasse zu einem Merkmalswerttupelbereich durch die Dateneingabeeinheit (4), wobei der Merkmalswerttupelbereich aus einem oder mehreren Merkmalswerttupeln gebildet wird,
f) Einteilung des Merkmalswerttupelbereichs in einen Kernbereich und einen Randbereich mittels Eingabe eines Konfidenzschwellenwertes, wobei eine Größe des Kernbereichs durch ein benutzergesteuertes Einstellen des Konfidenzschwellenwertes mittels der Dateneingabeeinheit (4) bestimmt wird,
und wobei in dem Kernbereich eine höhere Wahrscheinlichkeit einer korrekten Zuordnung des Merkmalswerttupels des Lebensmittelobjekts zu dem Merkmalswerttupelbereich gegeben ist als in dem Randbereich,
g) Ausgeben einer Erkennungsrate und einer Kernbereichszuordnungsrate als abhängige Größen des Konfidenzschwellenwertes durch die Datenausgabeeinheit (5),
h) automatisches Zuordnen des Merkmalswerttupels des Lebensmittelobjekts 1 zu dem Merkmalswerttupelbereich, wobei die Zuordnung entweder zu dem Kernbereich oder zu dem Randbereich erfolgt,
i) Ausgeben des Ergebnisses der Zuordnung des Merkmalswerttupels des Lebensmittelobjekts 1 zu dem Merkmalswerttupelbereich als Zuordnungsergebnis, unter Angabe der zugeordneten Klasse, durch die Datenausgabeeinheit (5), wobei die Ausgabe des Zuordnungsergebnisses mit einer Zuordnung zu dem Kernbereich, als Kernbereichszuordnung, oder mit einer Zuordnung zu dem Randbereich, als Randbereichszuordnung, erfolgt.

9. Verfahren nach Anspruch 8,
**dadurch gekennzeichnet,**
**dass** bei der Kernbereichszuordnung eine automatische Klassifikation erfolgt.

10. Verfahren nach Anspruch 8 oder 9,
**dadurch gekennzeichnet,**
**dass** bei einer Randbereichszuordnung eine Klassifikation als nicht oder nicht zuverlässig durchführbar ausgegeben wird.

11. Verfahren nach einem der Ansprüche 8 bis 10,
**dadurch gekennzeichnet,**
**dass** nach dem Verfahrensschritt i) ein zusätzlicher Verfahrensschritt j) ausgeführt wird, wobei in Verfahrensschritt j) eine benutzergesteuerte Überwachung und optionale Korrektur der automatischen Zuordnung des Merkmalswerttupels des Lebensmittelobjekts 1 zu dem Merkmalswerttupelbereich durchgeführt wird.

12. Verfahren nach einem der Ansprüche 8 bis 11,
**dadurch gekennzeichnet,**
**dass** bei einem Vorhandensein mehrerer Zuordnungsergebnisse unterschiedlicher Lebensmittelobjekte, die Zuordnungsergebnisse geordnet, in Abhängigkeit von einem Konfidenzmaß, ausgegeben werden.

13. Verfahren nach einem der Ansprüche 8 bis 12,
**dadurch gekennzeichnet,**
**dass** ein zusätzlicher Verfahrensschritt k) durchführbar ist, wobei in Verfahrensschritt k) ein Archivieren der optischen Daten und des zugehörigen Zuordnungsergebnisses erfolgt.

## Claims

1. An apparatus for classifying a food item (1) of organic or irregular structure,
having an image capture unit (2), an evaluation unit (3), a data input unit (4) and a data output unit (5), wherein the evaluation unit (3) is connected to the image capture unit (2), to the data input unit (3) and to the data output unit (4), and wherein the image capture unit (2) can be used to capture the food item (1) as optical data and to provide the optical data so as to be transmittable to the evaluation unit (3), and wherein the evaluation unit (3) can be used to extract feature values of the food item (1) from the optical data, wherein the feature values can be combined to form a feature value tuple for the food item (1) and wherein the feature value tuple of the food item (1) can be automatically assigned to a feature value tuple range, wherein the feature value tuple range is formed by one or more feature value tuples and wherein the feature value tuple range can be assigned a class, and wherein the data input unit (4) can be used to perform the assignment of the class to the feature value tuple range,
**characterized**
**in that** the evaluation unit (3) can be used to provide a categorization for the feature value tuple range into a core range and a marginal range, wherein in the core range there is a higher probability of correct assignment of the feature value tuple of the food item (1) to the feature value tuple range than in the marginal range,
and **in that** the categorization of the feature value tuple range can be stipulated by means of a confidence threshold value, wherein the confidence threshold value can stipulate a magnitude for the core range,
and **in that** the confidence threshold value can be set under user control by means of the data input unit (4), and in that the data output unit (5) can be used to output a recognition rate and a core range assignment rate, as dependent magnitudes of the confidence threshold value, and in that the automatic assignment of the feature value tuple of the food item to the feature value tuple range can be provided in a manner separated according to the core range and marginal range,
and **in that** the result of the assignment of the feature value tuple of the food item to the feature value tuple range can be output, specifying the assigned class, as an assignment result, with an assignment to the core range, as a core range assignment, or with an assignment to the marginal range, as a marginal range assignment.

2. The apparatus as claimed in claim 1,
**characterized**
**in that** the core range assignment can be used to provide an automatic classification.

3. The apparatus as claimed in claim 1 or 2,
**characterized**
**in that** the marginal range assignment can be used to output a classification as not or not reliably able to be performed.

4. The apparatus as claimed in one of the preceding claims,
**characterized**
**in that** the automatic assignment of the feature value tuple of the food item (1) to the feature value tuple range can be monitored and corrected under user control.

5. The apparatus as claimed in one of the preceding claims,
**characterized**
**in that** when there are a plurality of assignment results for different food items, it is possible to provide an output of the assignment results in organized fashion on the basis of a measure of confidence.

6. The apparatus as claimed in one of the preceding claims,
**characterized**
**in that** the optical data and the associated assignment result can be archived.

7. The apparatus as claimed in one of the preceding claims,
**characterized**
**in that** the image capture unit (2) can be used to capture the food item (1) as optical data on a transport system (10) and in that the assignment result can be provided in real time and in that the assignment result can be used to control external units.

8. A method for classifying a food item (1) of organic or irregular structure,
by means of an image capture unit (2), an evaluation unit (3), a data input unit (4) and a data output unit (5), wherein the evaluation unit (3) is connected to the image capture unit (2), to the data input unit (4) and to the data output unit (5), having the following method steps:
a) Capture of the food item (1) as optical data by the image capture unit (2),
b) Transmission of the optical data to the evaluation unit (3),
c) Extraction of feature values of the food item (1) from the optical data by the evaluation unit (3),
d) Combination of the feature values of the food item (1) to form a feature value tuple for the food item (1) by the evaluation unit (3),
e) Assignment of a class to a feature value tuple range by the data input unit (4), wherein the feature value tuple range is formed from one or more feature value tuples,
f) Categorization of the feature value tuple range into a core range and a marginal range by means of input of a confidence threshold value, wherein a magnitude of the core range is determined by user-controlled setting of the confidence threshold value by means of the data input unit (4), wherein in the core range there is a higher probability of correct assignment of the feature value tuple of the food item (1) to the feature value tuple range than in the marginal range,
g) Output of a recognition rate and a core range assignment rate as dependent magnitudes of the confidence threshold value by the data output unit (5),
h) Automatic assignment of the feature value tuple of the food item (1) to the feature value tuple range, wherein the assignment is made either to the core range or to the marginal range,
i) Output of the result of the assignment of the feature value tuple of the food item (1) to the feature value tuple range as an assignment result, specifying the assigned class, by the data output unit (5), wherein the assignment result is output with an assignment to the core range, as a core range assignment, or with an assignment to the marginal range, as a marginal range assignment.

9. The method as claimed in claim 8,
**characterized**
**in that** the core range assignment prompts an automatic classification to take place.

10. The method as claimed in claim 8 or 9,
**characterized**
**in that** a marginal range assignment prompts a classification to be output as not or not reliably able to be performed.

11. The method as claimed in one of claims 8 to 10,
**characterized**
**in that** after method step i) an additional method step j) is carried out, wherein method step j) involves user-controlled monitoring and optional correction of the automatic assignment of the feature value tuple of the food item (1) to the feature value tuple range being performed.

12. The method as claimed in one of claims 8 to 11,
**characterized**
**in that** when there are a plurality of assignment results for different food items the assignment results are output in organized fashion, on the basis of a measure of confidence.

13. The method as claimed in one of claims 8 to 12,
**characterized**
**in that** an additional method step k) can be performed, wherein method step k) involves the optical data and the associated assignment result being archived.

## Revendications

1. Dispositif pour la classification d'un objet alimentaire (1) de structure naturelle ou irrégulière,
comportant une unité de capture d'image (2), une unité d'évaluation (3), une unité d'entrée de données (4) et une unité de sortie de données (5), l'unité d'évaluation (3) étant relié à l'unité de capture d'image (2), à l'unité d'entrée de données (4) et à l'unité de sortie de données (5), et l'unité de capture d'image (2) permettant une capture de l'objet produit alimentaire (1) sous forme de données optiques, lesquelles sont mises à disposition pour pouvoir être transférées à l'unité d'évaluation (3), et l'unité d'évaluation (3) permettant l'extraction de valeurs caractéristiques de l'objet alimentaire (1) à partir des données optiques, lesquelles valeurs caractéristiques peuvent être regroupées en un n-uplet de valeurs caractéristiques de l'objet alimentaire (1), et le n-uplet de l'objet alimentaire (1) pouvant être classé automatiquement dans une plage de n-uplets de valeurs caractéristiques, ladite plage de n-uplets de valeurs caractéristiques étant formée par un ou plusieurs n-uplets de valeurs caractéristiques, une classe pouvant en outre être attribuée à ladite plage de n-uplets de valeurs caractéristiques, ladite attribution de classe à une plage de n-uplets de valeurs caractéristiques pouvant être réalisée au moyen de l'unité d'entrée de données (4),
est **caractérisée en ce**
**que** la séparation de la plage de n-uplets de valeurs caractéristiques entre une région coeur et une région marginale par l'unité d'évaluation (3) est permise, et en ce que le classement du n-uplet de valeurs caractéristiques de l'objet alimentaire (1) peut être réalisée séparément en fonction de la région coeur et de la région marginale et en ce que dans la région coeur la probabilité d'une attribution correcte du n-uplet de valeurs caractéristiques de l'objet alimentaire (1) à la plage de n-uplets de valeurs caractéristiques est plus élevée que dans la région marginale,
et en ce que la séparation de la plage de n-uplets de valeurs caractéristiques peut être fixée au moyen d'une valeur seuil de confiance, la taille de la région coeur pouvant être définie par ladite valeur seuil de confiance,
et en ce que la valeur seuil de confiance peut être réglée par l'utilisateur au moyen de l'unité d'entrée de données (4),
et en ce que l'unité de sortie de données (5) peut être utilisée pour émettre un taux de reconnaissance et un taux d'attribution de la région coeur comme grandeur dépendante de la valeur seuil de confiance, et en ce que l'attribution automatique du n-uplet de valeurs caractéristiques de l'objet alimentaire à la plage de n-uplets de valeurs caractéristiques peut être fournie séparément en fonction de la région coeur et la région marginale,
et en ce que le résultat de l'attribution du n-uplet de valeurs caractéristiques de l'objet alimentaire à la plage de n-uplets de valeurs caractéristiques peut être émis en indiquant la classe attribuée comme résultat d'attribution avec une attribution à la région coeur comme attribution de la région coeur ou avec une attribution à la région marginale comme attribution de la région marginale.

2. Dispositif suivant la revendication 1
est **caractérisée en ce**
**que** l'attribution de la région coeur permet la classification automatique.

3. Dispositif suivant les revendications 1 ou 2
est **caractérisée en ce**
**qu'**une classification peut être émis comme non réalisable ou non réalisable avec fiabilité au moyen de l'attribution de la région marginale.

4. Dispositif suivant une des revendications précédentes
est **caractérisée en ce**
**que** l'attribution automatique du n-uplet de valeurs caractéristiques de l'objet alimentaire à la plage de n-uplets de valeurs caractéristiques peut être surveillée et corrigée par l'utilisateur.

5. Dispositif suivant une des revendications précédentes
est **caractérisée en ce**
**qu'**en cas de présence de plusieurs résultats d'attribution de différents objets alimentaires, l'émission des résultats d'attribution peut être effectuée de manière ordonnée suivant une valeur de confiance.

6. Dispositif suivant une des revendications précédentes
est **caractérisée en ce**
**que** les données optiques et le résultat appartenant d'attribution peuvent être archivés.

7. Dispositif suivant une des revendications précédentes
est **caractérisée en ce**
**que** l'objet alimentaire (1) peut être saisi sous forme de données optiques sur un système de transport (10) au moyen de l'unité de capture d'image (2) et que le résultat d'attribution peut être fourni en temps réel et que des unités externes peuvent être commandées au moyen du résultat d'attribution.

8. Procédé pour la classification d'un objet alimentaire (1) de structure naturelle ou irrégulière,
au moyen d'une unité de capture d'image (2), une unité d'évaluation (3), une unité d'entrée de données (4) et une unité de sortie de données (5), l'unité d'évaluation (3) étant reliée à l'unité de capture d'image (2), l'unité d'entrée de données (4) et l'unité de sortie de données (5), comportant les suivantes étapes de procédé:
a) Saisie d'un objet alimentaire 1 sous forme de données optiques par l'unité de capture d'image (2),
b) Transmission des données optiques à l'unité d'évaluation (3),
c) Extraction de valeurs caractéristiques de l'objet alimentaire 1 à partir des données optiques par l'unité d'évaluation (3),
d) Regroupement des valeurs caractéristiques de l'objet alimentaire 1 pour former un n-uplet de valeurs caractéristiques de l'objet alimentaire 1 par l'unité d'évaluation (3),
e) Attribution d'une classe à une plage de n-uplets de valeurs caractéristiques par l'unité d'entrée de données (4), ladite plage de n-uplets de valeurs caractéristiques étant formée d'un ou plusieurs n-uplets de valeurs caractéristiques,
f) Séparation de la plage de n-uplets de valeurs caractéristiques en une région coeur et une région marginale en entrant une valeur seuil de confiance, la taille de la région coeur étant définie par le réglage de l'utilisateur de la valeur seuil de confiance au moyen de l'unité d'entrée de données (4),
et dans la région coeur la probabilité d'une attribution correcte du n-uplet de valeurs caractéristiques de l'objet alimentaire (1) à la plage de n-uplets de valeurs caractéristiques étant plus élevée que dans la région marginale,
g) Émission d'un taux de reconnaissance et d'un taux d'attribution de la région coeur comme grandeur dépendante de la valeur seuil de confiance par l'unité de sortie de données (5),
h) Attribution automatique du n-uplet de valeurs caractéristiques de l'objet alimentaire 1 à la plage de n-uplets de valeurs, l'attribution étant effectuée soit à la région coeur, soit à la région marginale,
i) Émission du résultat de l'attribution du n-uplet de valeurs caractéristiques de l'objet alimentaire 1 à la plage de n-uplets de valeurs caractéristiques comme résultat d'attribution en indiquant la classe attribuée par l'unité de sortie de données (5), l'émission du résultat de l'attribution étant effectuée avec une attribution à la région coeur comme attribution de la région coeur ou avec une attribution à la région marginale comme attribution de la région marginale.

9. Procédé suivant la revendication 8
est **caractérisée en ce**
**qu'**une classification automatique est effectuée pendant l'attribution de la région coeur.

10. Procédé suivant les revendications 8 ou 9
est **caractérisée en ce**
**qu'**une classification peut être émis comme non réalisable ou non réalisable avec fiabilité lors d'une attribution à la région marginale.

11. Procédé suivant une ou plusieurs des revendications 8 à 10
est **caractérisée en ce**
**qu'**une étape de procédé supplémentaire j) est effectuée après l'étape de procédé i) et que pendant l'étape de procédé j) une surveillance commandée par l'utilisateur et une correction facultative de l'attribution automatique du n-uplet de valeurs caractéristiques de l'objet alimentaire 1 à la plage de n-uplets de valeurs sont effectuées.

12. Procédé suivant une ou plusieurs des revendications 8 à 11
est **caractérisée en ce**
**qu'**en cas de présence de plusieurs résultats d'attribution de différents objets alimentaires, les résultats d'attribution sont émis de manière ordonnée suivant une valeur de confiance.

13. Procédé suivant une ou plusieurs des revendications 8 à 12
est **caractérisée en ce**
**qu'**une étape de procédé supplémentaire k) peut être effectuée et que pendant cette étape de procédé k) l'archivage des données optiques et du résultat d'attribution appartenant est effectué.
